# EUROPEAN PATENT APPLICATION

(11) **EP 4 818 192 A1**
(43) Date of publication of application: **07.10.2026**
(21) Application number: 24898133.4
(22) Date of filing: 27.11.2024
(51) Int. Cl.: C07K 7/08, A61P 1/02, A61P 21/00, A61K 38/00

(54) **USE OF GRP78-LIKE PEPTIDES**

(30) Priority: 28.11.2023 KR 20230167420
(71) Applicant: Chungbuk National University Industry-Academic Cooperation Foundation, Cheongju-si, Chungcheongbuk-do 28644 (KR); Seoul National University R&DB Foundation, Seoul 08826 (KR); Nibec Co., Ltd., Chungcheongbuk-do 27816 (KR)
(72) Inventor: CHUNG, Chong-Pyoung, Seoul 05618 (KR); PARK, Yoon Jeong, Seoul 08291 (KR); LEE, Jue-Yeon, Gwacheon-si Gyeonggi-do 13831 (KR); LEE, Dong Woo, Seoul 08213 (KR); JO, Beom Soo, Seoul 05817 (KR); PARK, Yoon Shin, Cheongju-si Chungcheongbuk-do 28415 (KR); CHOI, Da Hyeon, Cheongju-si Chungcheongbuk-do 28644 (KR); LEE, Kyeong Eun, Cheongju-si Chungcheongbuk-do 28644 (KR)
(74) Representative: Mewburn Ellis LLP
(86) International application number: PCT/KR2024/019031
(87) International publication number: WO 2025/116539

(57) **Abstract**

The present invention relates to a use of GRP78, and a similar peptide thereof or a nucleic acid encoding same. The peptide or nucleic acid according to the present invention can increase the expression of a biomarker having reduced expression in senescent cells, and thus is useful for restoring functions, and the like, of muscle tissue of which functions are reduced by aging.

## Description

### Technical Field

The present invention relates to use of GRP78 and a GRP78-like peptide or a nucleic acid encoding the same, and more particularly, to a composition comprising GRP78 and a GRP78-like peptide or a nucleic acid encoding the same for inhibiting or reversing aging of cells, tissues or organs, or restoring or regenerating the function of aged or damaged cells, tissues or organs, a pharmaceutical composition comprising GRP78 and a GRP78-like peptide or a nucleic acid encoding the same for preventing or treating muscle diseases, and a cosmetic composition comprising GRP78 and a GRP78-like peptide or a nucleic acid encoding the same for preventing or reversing aging.

### Background Art

Aging refers to gradual deterioration of the structure and functions of the body with age. First, differentiation and proliferation decrease at the cellular level, leading to changes in the structure of specific molecules and altered response pathways. This, in turn, leads to inevitable anatomical and physiological changes that extend beyond the cellular and molecular levels. Homeostasis in organs and systems deteriorates, dramatically increasing susceptibility to external stress, disease, and death (Cynthia J Kenyon, Nature. 2010 Mar 25;464(7288):504-12).

Currently, developed countries around the world, including Korea, are facing rapid social structural changes due to an aging population. Beyond passive responses to aging, it is crucial to understand the biological principles underlying the disease phenomenon of aging and, furthermore, to develop new technologies to control and reverse aging. The World Health Organization (WHO) categorized aging as a preventable and treatable disease, for example, assigned a disease code to aging in the disease statistical classification of ICD-11 (International Classification of Diseases-11) in 2018.

Telomeres are DNA terminal regions located at the ends of chromosomes consisting of DNA and proteins, and serve to protect the ends of chromosomes. When a parent cell divides to produce daughter cells, there is a limit to how completely the ends of the DNA molecule can be replicated. This leads to gradual loss of telomeres with each cell division. This phenomenon is specific to aging, and telomeres will continue to shorten with each cell division unless other mechanisms exist to prevent telomere attrition.

When telomeres shorten below a predetermined length, cells reach senescence and stop dividing, resulting in cell death, which accelerates human aging (Saliha Rizvi, et al., Curr Aging Sci. 2014;7(3):161-7). In humans, a deficiency of telomerase, which is involved in telomere end formation, may cause various diseases, such as pulmonary fibrosis, dyskeratosis congenita, and aplastic anemia. These diseases are attributed to a lack of the ability of specific tissues to regenerate (Joao Pedro de Magalhaes, Joao F Passos, Mech Ageing Dev. 2018 Mar:170:2-9). The ends of long, connected genomes can be broken, and when cells recognize these broken ends as DNA damage, the DNA repair system may cause incorrect genome ligation. To prevent this phenomenon, telomeres are protected by a protein complex called "shelterin" which binds to the ends of DNA. Even if telomeres do not shorten, problems with this protective structure may affect cellular aging and survival. In some cases, problems with shelterin may damage the telomere protective structure and lead to chromosomes misaligning. These problems may also reduce tissue resilience, accelerate aging, and impact cell survival. The causal relationship between telomere loss, cellular aging, and subject-level aging has been studied through genetic modification experiments using animal models. In particular, mice engineered to have shorter or longer telomeres had shorter or longer lifespans, respectively (Bruno Bernardes de Jesus, et al., EMBO Mol Med. 2012 Aug;4(8):691-704).

Telomere length-related factor-1 (TRF-1) encodes a telomere-specific protein that regulates telomere length and acts as a telomerase inhibitor and the expression thereof is reduced in aged cells (Keiji Okamoto et al., J Biol Chem. 2008 Aug 29;283(35):23981-8). Telomere length-related factor-2 (TRF-2) is present at telomeres, forms the shelterin nucleoprotein complex, and plays a role in telomere length regulation, and expression of TRF-2 is reduced in aged cells (Giacomo Buscemi et al., Curr Biol. 2009 May 26;19(10):874-9). Protection of Telomeres 1 (POT1) encodes a nuclear protein involved in telomere maintenance and binds to TTAGGG repeats to regulate telomere length, and the expression thereof is reduced in aged cells (Tomas Aramburu, et al., Comput Struct Biotechnol J. 2020 Jul 3:18:1939-1946). Human telomerase reverse transcriptase (hTERT) is a human telomerase reverse transcriptase and is a component of the telomerase complex, and the expression thereof is reduced in aged cells (Anastasia I Palamarchuk, et al., Biomedicines. 2023 Apr 4;11(4):1091). Glucose-regulated protein 78 (GRP78) plays a role in maintaining protein folding and state when newly synthesized proteins are translocated to the endoplasmic reticulum. GRP78 is abundant on the cell surface in young cells, but is abundant in the nucleus in aged cells and expression thereof decreases (Da Hyeon Choi, et al., Biomaterials. 2021 Nov:278:121156).

Accordingly, as a result of extensive research to discover novel substances capable of reversing the transformation of aged cells into young cells, the present inventors have discovered peptides that have the effect of increasing biomarkers whose expression is reduced in aged cells. Surprisingly, when GRP78-like peptides are expressed in aged cells, the expression pattern of aged biomarkers changes to that of young cells. Furthermore, when GRP78-like peptides are administered to aged mice, the present inventors have found that the expression pattern of aging-related biomarkers in aged tissues changes to the expression pattern of young tissues and tissue function is restored. Based on the findings, the present invention was completed.

The information disclosed in this Background section is provided only for better understanding of the background of the present invention, and therefore it may not include information that forms the prior art that is already obvious to those skilled in the art.

### [Prior Art Literature]

(Non-patent Literature 1) Cynthia J Kenyon, Nature. 2010 Mar 25;464(7288):504-12
(Non-patent Literature 2) Da Hyeon Choi, et al., Biomaterials. 2021 Nov:278:121156

### Summary of the Invention

It is one object of the present invention to provide a peptide capable of inhibiting or reversing aging and restoring or regenerating the functions of cells, tissues, or organs associated with aging by increasing the expression of biomarkers whose expression is reduced in aged cells, and use of the peptide.

In accordance with the present invention, the above and other objects can be accomplished by the provision of a composition comprising GRP78, a GRP78-like peptide, or a nucleic acid encoding the same as an active ingredient, for inhibiting or reversing aging in cells, tissues, or organs, or restoring or regenerating the functions of aged or damaged cells, tissues, or organs.

### Brief Description of Drawings

FIG. 1 shows plasmid maps of a GRP78 expression vector and a control vector.
FIG. 2 shows the GRP78 mRNA expression levels of different cells transfected with the GRP78 expression vector, wherein NT represents a non-treated experimental group, Mock represents an experimental group transfected with the control vector alone, and GRP78 represents an experimental group transfected with the GRP78 expression vector.
FIG. 3 shows the results of confocal microscopy after transfection of cells (young and aged cells) with the vector carrying GRP78-EGFP, wherein NT represents a non-treated experimental group, Mock represents an experimental group transfected with the control vector alone, and GRP78 represents an experimental group transfected with the GRP78 expression vector (blue: nucleus, red: F-actin, green: GRP78-EGFP).
FIG. 4 shows a cluster proportion (percentage) of cells expressing GRP78-EGFP in young and aged cells transfected with the GRP78-EGFP expression vector, measured using FACS.
FIG. 5 is a graph showing the changes in mRNA expression of TERT, a factor involved in telomere length, when GRP78 was expressed in dental pulp-derived stem cells (DPSCs and PDLSCs), wherein NT represents a non-treated experimental group, Mock represents an experimental group transfected with the control vector alone, and GRP78 represents an experimental group transduced with the GRP expression vector (*p<0.05, **p<0.01).
FIG. 6 illustrates an overall process of measuring the histological changes in muscle when the GRP78 expression vector is introduced into the muscles of mice of each age group.
FIG. 7 is a confocal micrograph showing the expression of GRP78, as observed by fluorescence, after the GRP78-EGFP expression vector was injected into the muscle of a mouse.
FIG. 8 is a graph showing the results of GRP78 mRNA expression in mice of each age group when the GRP78 expression vector was introduced into mice of each age group (****p<0.0001).
FIG. 9 shows the results of Western blotting to determine the level of GRP78 protein expression in tissues of mice of each age group.
FIG. 10 shows comparison of changes in muscle bundle thickness when GRP78 was expressed in mice of each age group.

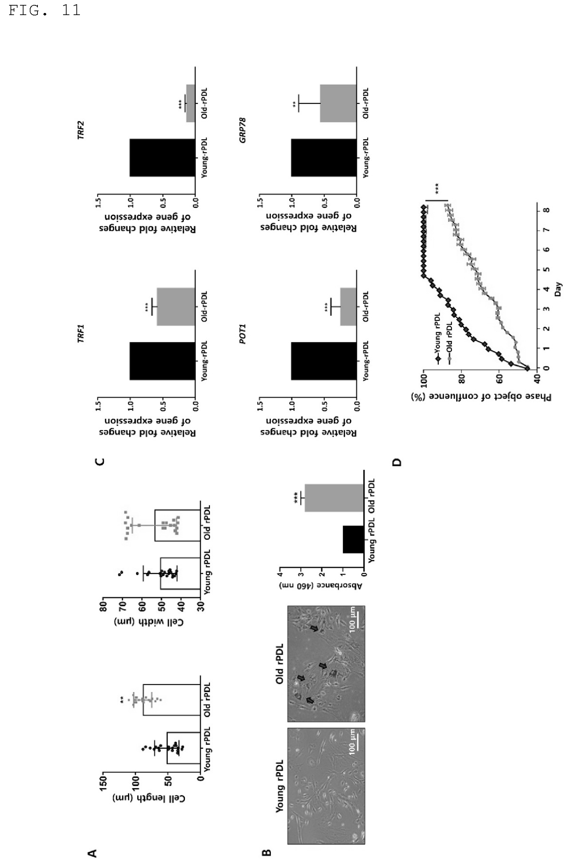

FIG. 11 shows the changes in cell characteristics with age in periodontal ligament tissue-derived cells of young rats (11 weeks old, control group) and aged rats (96 weeks old, aged group). FIG. 11A shows the cell length and width of the control group and aged group (**p<0.01). FIG. 11B shows the absorbance measured using the SA-β gal staining method to determine the expression of beta-galactosidase, which is highly expressed in aged cells (***p<0.001). FIG. 11C shows the gene expression levels of aging-related biomarkers, telomere length related factor-1 (TRF-1), telomere length related factor-2 (TRF-2), protection of telomeres 1 (POT1), and glucose-regulated protein 78 (GRP78), measured by RT-PCR (**p<0.01, ***p<0.001). FIG. 11D shows the cell proliferation (***p<0.001).
FIG. 12 is a confocal micrograph showing the cell permeability of the peptides of SEQ ID NOs: 1 to 4. FIG. 12A shows the cell permeability of young rPDL and FIG. 12B shows the cell permeability of old rPDL. Sequence 1 represents a group treated with the peptide of SEQ ID NO: 1, Sequence 2 represents a group treated with the peptide of SEQ ID NO: 2, Sequence 3 represents a group treated with the peptide of SEQ ID NO: 3, and Sequence 4 represents a group treated with the peptide of SEQ ID NO: 4. The above descriptions also apply to the subsequent figures.

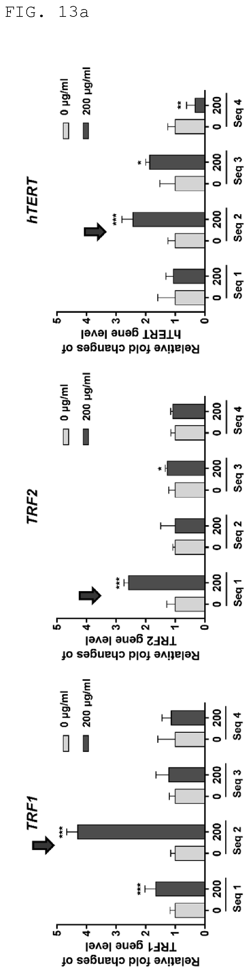

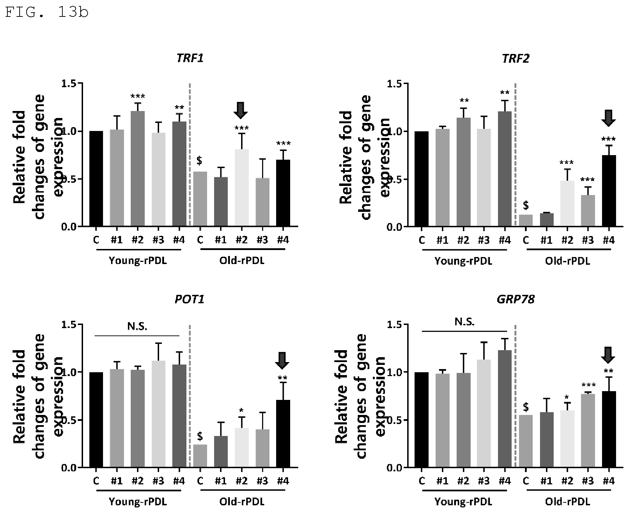

FIG. 13 shows the changes in the expression of aging-related biomarkers induced by the peptides of SEQ ID NOs: 1 to 4. FIG. 13A shows the changes in the expression of aging-related biomarkers TRF-1, TRF-2, and human telomerase reverse transcriptase (hTERT) by peptides of sequence numbers 1 to 4 in human tonsil tissue-derived stem cells (20 passages) aged by continuous culture, measured by RT-PCR (*p<0.05, **p<0.01, ***p<0.001), FIG. 13B shows the changes in the expression of aging-related biomarker genes TRF-1, TRF-2, POT1, and GRP78 by peptides of SEQ ID NOS: 1 to 4 in periodontal ligament tissue-derived cells extracted from young rats (12 weeks old) and aged rats (96 weeks old), measured by RT-PCR ($p<0.001, comparison between Young rPDL_control and Old rPDL_control, p<0.001, *** p<0.001, ** p<0.01, *p<0.05, compared with the control group when treated with the peptide). Seq 1 represents a group treated with the peptide of SEQ ID NO: 1, Seq 2 represents a group treated with the peptide of SEQ ID NO: 2, Seq 3 represents a group treated with the peptide of SEQ ID NO: 3, and Seq 4 represents a treated with the peptide of SEQ ID NO: 4. The above descriptions also apply to the subsequent figures.
FIG. 14 shows the time it took for 8-, 48-, and 72-week-old mice to remain on a rotating rod without falling. NT represents a non-treated experimental group, sequence 3 represents a group treated with the peptide of SEQ ID NO: 3, and sequence 4 represents a group treated with the peptide of SEQ ID NO: 4.
FIG. 15 shows a peptide administration schedule for mice of each age group. Seq 19 represents a muscle regeneration peptide of SEQ ID NO: 19, and the above descriptions also apply to the subsequent figures.
FIG. 16 shows the results of H&E staining and muscle fiber diameter measurements of mouse muscle tissue administered the peptide of SEQ ID NO: 3 or SEQ ID NO: 4 for 4 weeks.
FIG. 17 shows the results of H&E staining and muscle fiber diameter measurements of mouse muscle tissues administered the peptide of SEQ ID NO: 3 or SEQ ID NO: 4 for 4 weeks, followed by an additional 4-week administration of peptides of SEQ ID NO: 3 or SEQ ID NO: 4 alone, or in combination with a muscle regeneration peptide (SEQ ID NO: 19) (*p<0.05, **p<0.01).
FIG. 18 shows the results of confocal microscopy and then fluorescence intensity measurements to determine MyoD1 expression in muscle tissues of mice administered peptides of SEQ ID NO: 3 or SEQ ID NO: 4 for 4 weeks (**p<0.01, ****p<0.0001).
FIG. 19 shows the results of confocal microscopy and then fluorescence intensity measurements to determine MYH expression in muscle tissue of mice administered the peptide of SEQ ID NO: 3 and SEQ ID NO: 4 for 4 weeks (***p<0.001, ****p<0.0001).
FIG. 20 shows the results of confocal microscopy and then fluorescence intensity measurements to determine MyoD1 expression in muscle of mice administered the peptide of SEQ ID NO: 3 or SEQ ID NO: 4 for 4 weeks, followed by an additional 4 weeks of administration of peptides of SEQ ID NO: 3 or SEQ ID NO: 4 alone, or in combination with a muscle regeneration peptide (SEQ ID NO: 19) (*p<0.05, ***p<0.001, ****p<0.0001).
FIG. 21 shows the results of confocal microscopy and fluorescence intensity measurements of MYH expression in the muscles of mice injected with the peptide of SEQ ID NO: 3 or SEQ ID NO: 4 for 4 weeks, followed by an additional 4 weeks of administration of peptides of SEQ ID NO: 3 or SEQ ID NO: 4 alone, or in combination with a muscle regeneration peptide (SEQ ID NO: 19) (****p<0.0001).
FIG. 22A is a graph showing changes in human telomerase gene expression when young and aged TMSC cells were treated with a GRP78-like peptide.
FIG. 22B shows the comparison of telomerase activity in young and aged cells using the TRAP assay for visual comparison in a length of telomerase, which is an enzyme that elongates telomeres (***p<0.001).
FIG. 22C shows the comparison in telomere length, which is a representative marker of aging, using TRF assay for measuring TRF length in young and aged cells (*p<0.05).
FIG. 22D shows the results of Southern blotting to determine the visible telomere length in young and aged cells.
FIG. 23A shows the results of changes in mitochondrial protein expression after treatment of young and aged TMSC cells with a fluorescently labeled GRP78-like peptide (Rhodamine B).
FIG. 23B is a graph showing the results of quantitative analysis of the protein expression shown in FIG. 12A using ImageJ.
FIG. 23C is a graph showing the changes in mitochondrial DNA (mtDNA) copy number measured using qRT-PCR to determine quantitative changes in mtDNA.
FIG. 23D shows the changes in protein expression of factors related to mitochondrial activity and biogenesis in young and aged cells (*p<0.05, **p<0.01, ***p<0.001).
FIG. 23E shows the results of changes in gene and protein expression induced by GRP78-like peptides of PPARγ, PGC1α, and p53, which are factors associated with mitochondria and telomeres (**p<0.01, ****p<0.0001).

### Detailed Description and Preferred Embodiments of the Invention

Unless defined otherwise, all technical and scientific terms used herein have the same meanings as appreciated by those skilled in the field to which the present invention pertains. In general, the nomenclature used herein is well-known in the art and is ordinarily used.

As used herein, the term "aging" refers to the decline in cell differentiation or proliferation capacity due to increasing age, as well as changes in bodily function and condition that occur over time. While the onset of aging varies from individual to individual, aging typically refers to the decline in normal function of cells, tissues, or organs over time. Aging progresses independently of disease. Aging may increase the incidence of disease and may accelerate the functional decline of damaged cells, tissues, or organs in diseased subjects.

In an *in vitro* experimental setting, the term "aging" may refer to the decline in cell differentiation or proliferation capacity due to increased number of passages. As used herein, the term "passage" refers to replacing a culture vessel or culturing in each cell population in a process of periodically transferring a part of cells to a new culture vessel and then replacing the culture medium with fresh one to continue culturing the cells over time in a healthy state. Single culture vessel replacement or culture in each cell population is referred to as "one passage".

In one embodiment of the present invention, the expression of aging-related biomarkers TRF-1, TRF-2, POT1, hTERT, and GRP78 genes decreased in aged periodontal ligament (PDL) cells and tonsil-derived mesenchymal stem cells (TMSC). Furthermore, treatment of aged cells with a GRP78-like peptide according to the present invention resulted in a re-increase in the expression of these decreased biomarker genes. Furthermore, the result of administration of the GRP78-like peptide according to the present invention to a natural aging mouse model showed the restoration of muscle function in aged mice. Furthermore, administration of the GRP78-like peptide according to the present invention to aged TMSC resulted in the restoration of expression of factors related to telomere length and mitochondrial activity. These were surprising effects that were completely unexpected in the prior art, separate from the utility of GRP78 as an aging-related biomarker.

Accordingly, in one aspect, the present invention is directed to a composition comprising GRP78, a GRP78-like peptide, or a nucleic acid encoding the same as an active ingredient, for inhibiting or reversing aging in cells, tissues, or organs, or restoring or regenerating the functions of aged or damaged cells, tissues, or organs.

GRP78 refers to a 78-kDa glucose-regulated protein and is an endoplasmic reticulum (ER) chaperone protein, also known as BiP or HSPA5. GRP78 is known not only for its role in protein folding but also as a crucial factor in regulating the unfolded protein response (UPR). Under endoplasmic reticulum homeostasis, GRP78 normally binds to three UPR transmembrane sensors, i.e., ATF6, PERK, and IRE1, and maintains the sensors in an inactive state. Under ER stress conditions, where unfolded proteins accumulate in the ER lumen, GRP78 is isolated from the UPR sensors and contributes to the activation thereof. Activated UPR alleviates ER stress by reducing protein translation and increasing ER folding capacity. In this process, GRP78 is upregulated. When ER homeostasis is not restored, the UPR may induce apoptosis.

GRP78 acts in a diverse range of protein folding processes through two domains. One is the nucleotide binding domain (NBD), which has the activity of binding to ATP and hydrolyzing ATP, and the other is the substrate binding domain (SBD), which binds to substrate proteins. The NBD consists of two subdomains (I and II), which are each further divided into two smaller subdomains (A and B). These subdomains are separated by a cleft of an area where a nucleotide is bound, together with one Mg²⁺ ion and two K⁺ ions. The SBD is also divided into two subdomains (alpha and beta). The SBD alpha subdomain provides a pocket for substrate protein binding and the SBD beta subdomain acts as a lid covering the binding pocket.

GRP78 activity is regulated by an allosteric ATPase cycle. When ATP binds to the NBD, the lid (SBD beta subdomain) opens, inducing a conformational change in the SBD that reduces the binding affinity of GRP78 to the substrate protein. When ATP is hydrolyzed, ADP binds to the NBD, closing the lid on the bound substrate. This increases the binding affinity of substrate protein and prevents the bound substrate protein from folding improperly. When ADP is replaced by ATP, the lid opens again, releasing the substrate protein. This ATPase cycle is tightly regulated by nucleotide binding and substrate protein binding. Substrate protein binding promotes ATP hydrolysis, while nucleotide binding influences either substrate protein binding or release.

In the present invention, the amino acid sequence of GRP78 and the base sequence encoding the same may be obtained from a known database such as NCBI's GenBank (e.g., GenBank Accession AAA52614.1).

### GRP78 genetic base sequence (M19645.1 Human 78 kD glucose-regulated protein (GRP78) gene, complete CDs, source 1,5470 bp) (SEQ ID NO: 20):

CCCGGGGTCACTCCTGCTGGACCTACTCCGACCCCCTAGGCCGGGAGTGAAGGCGG
GACTTGTGCGGTTACCAGCGGAAATGCCTCGGGGTCAGAAGTCGCAGGAGAGATAGACAGC
TGCTGAACCAATGGGACCAGCGGATGGGGCGGATGTTATCTACCATTGGTGAACGTTAGAA
ACGAATAGCAGCCAATGAATCAGCTGGGGGGGCGGAGCAGTGACGTTTATTGCGGAGGGGG
CCGCTTCGAATCGGCGGCGGCCAGCTTGGTGGCCTGGGCCAATGAACGGCCTCCAACGAGC
AGGGCCTTCACCAATCGGCGGCCTCCACGACGGGGCTGGGGGAGGGTATATAAGCCGAGTA
GGCGACGGTGAGGTCGACGCCGGCCAAGACAGCACAGACAGATTGACCTATTGGGGTGTTT
CGCGAGTGTGAGAGGGAAGCGCCGCGGCCTGTATTTCTAGACCTGCCCTTCGCCTGGTTCG
TGGCGCCTTGTGACCCCGGGCCCCTGCCGCCTGCAAGTCGAAATTGCGCTGTGCTCCTGTG
CTACGGCCTGTGGCTGGACTGCCTGCTGCTGCCCAACTGGCTGGCAAGATGAAGCTCTCCC
TGGTGGCCGCGATGCTGCTGCTGCTCAGCGCGGCGCGGGCCGAGGAGGAGGACAAGAAGGA
GGACGTGGGCACGGTGGTCGGCATCGACTTGGGGACCACCTACTCCTGGTAAGTGGGGTTG
CGGATGAGGGGGACGGGGCGTGGCGCTGGCTGGCGTGAGAAGTGCGGTGCTGATGTCCCTC
TGTCGGGTTTTTGCAGCGTCGGCGTGTTCAAGAACGGCCGCGTGGAGATCATCGCCAACGA
TCAGGGCAACCGCATCACGCCGTCCTATGTCGCCTTCACTCCTGAAGGGGAACGTCTGATT
GGCGATGCCGCCAAGAACCAGCTCACCTCCAACCCCGAGAACACGGTCTTTGACGCCAAGC
GGCTCATCGGCCGCACGTGGAATGACCCGTCTGTGCAGCAGGACATCAAGTTCTTGCCGTT
CAAGGTTCGACCGGTTTTCCTCATCCAGTTAGAGAACGGGTGGGTGGTGGAGTATTTAGA
GTTATAAGTCTCTGGAAAAGTGTTGAGACAACAGTTGAAGGTTATAGACATGATGTATGTA
ATAACTTTAATACTATTAGTATGTTACAAAACTTAAGACAGTTGCTGTCGTACTGTCTACG
ATAGTTTAGGAATAAAAGACCGATTAAAACTGAACTTTGTAAGACACCTATACTCCCTGAA

GTATTTCTAGTCAATTTGCAGCCCCAAGGACCAAAATAAACCAAATTGTGGGGATGGTAG
TGGGTCTTTTAAACTTTGAGATGTCATTGTATCTGTGTCTGAAAACAATAATTCTTTAAAA
TAGGTGGTTGAAAAGAAAACTAAACCATACATTCAAGTTGATATTGGAGGTGGGCAAACAA
AGACATTTGCTCCTGAAGAAATTTCTGCCATGGTTCTCACTAAAATGAAAGAAACCGCTGA
GGCTTATTTGGGAAAGAAGGTAAATATTTCTAGAACAATGTTAAGTATTTTTTGATCATTA
GTATTCTCGGTTGGCTGTTATGTATAGAAGCCTTCGTGAAGGGTTTCAAAAATTTTAATCA
GAATGGTATTCATGCTTGTCACGGTTTAATTATTGAGTCCCTTTACTATAAGCCAAACAAA
AATAGACTTTTCATGTATTATTTAATGCTTACAATTCCAGGAACAATAAAATTTTATATGT
TGTATTCATCAATAATTGGCTTAAAAACTAAAGTGATGGTTTGACTGTAATTTTTTTTTTT
TGAGATGGAGTCTTGCTCTGTTGCCCAGGCTGGACTGCAGTGGCACGATCTCAGCTCACTG
CAACCTCTGCCTCCCGGGTTAAGCAGCTCTCCTGCCTCAGCCTCCAAGTAATGGAACGACA
GGCACACCACCACAGCTGGCTAATTTTTTTTTTTTTTTTAATTTTCAGTAGAGACAGGGT
TTCTCCACATTGCCAGGCTGGTCTTGAAATCCTGCCCTCAGGTTGATCCTCCTGCCTAGCC
TCCCAAAGTGCTGGATTATAGGCAGAAGCCACCGCCTGGCCAGACTGTAATTTAAATAAGG
GTTAAACTATGTGACAATACACTTAATTATCTTTATCCTTTTAGGTTACCCATGCAGTTGT
TACTGTACCAGCCTATTTTAATGATGCCCAACGCCAAGCAACCAAAGACGCTGGAACTATT
GCTGGCCTAAATGTTATGAGGATCATCAACGAGCCGTAAGTATGAAATTCAGGGATACGGC
ATATTTGCCAAATAGTGGAAATGTGAAGTACTGACAAAACTTTTCCCTTTTTCAATCTAAT
AGTACGGCAGCTGCTATTGCTTATGGCCTGGATAAGAGGGAGGGGGAGAAGAACATCCTGG
TGTTTGACCTGGGTGGCGGAACCTTCGATGTGTCTCTTCTCACCATTGACAATGGTGTCTT
CGAAGTTGTGGCCACTAATGGAGATACTCATCTGGGTGGAGAAGACTTTGACCAGCGTGTC
ATGGAACACTTCATCAAACTGTACAAAAAGAAGACGGGCAAAGATGTCAGGAAGGACAATA
GAGCTGTGCAGAAACTCCGGCGCGAGGTAGAAAAGGCCAAGGCCCTGTCTTCTCAGCATCA
AGCAAGAATTGAAATTGAGTCCTTCTATGAAGGAGAAGACTTTTCTGAGACCCTGACTCGG
GCCAAATTTGAAGAGCTCAACATGGTATGTTCCTTGTTTTCTGCTTTGCTAATGAGATCTC
CTTAGACTCTGAATTCAGGACATTGCATCTAGATACTTAGATAACAGACATCACAGTAACC

ATGTCTTTTTTCTAGGATCTGTTCCGGTCTACTATGAAGCCCGTCCAGAAAGTGTTGGAAG
ATTCTGATTTGAAGAAGTCTGATATTGATGAAATTGTTCTTGTTGGTGGCTCGACTCGAAT
TCCAAAGATTCAGCAACTGGTTAAAGAGTTCTTCAATGGCAAGGAACCATCCCGTGGCATA
AACCCAGATGAAGCTGTAGCGTATGGTGCTGCTGTCCAGGCTGGTGTGCTCTCTGGTGATC
AAGATACAGGTAGGTCATCATCGCAGCATCTTTCTTAGTGATTCAGTAGCTTGATGGAAGA
GCTCGGTACCCCTATTGCTTTAGAAAATACCAGAATATGAGCAACAAGGTCACACAGCTAG
TAAAGGGTATAAGTGAAGACAAGACTGGGGTAGTCTCCAAGATCATTAGCAACTGTTTAAT
TCACTGCCTTTAAAATGTGTGTGTTAGAACCTAACCAAATGTTAGAGAGATAAACTTTACA
TAGCTCATAGGGAGAACTTGAATTAAAAGTTAAATAACTTATCCTTACAGGTGACCTGGTA
CTGCTTCATGTATGTCCCCTTACACTTGGTATTGAAACTGTAGGAGGTGTCATGACCAAAC
TGATTCCAAGTAATACAGTGGTGCCTACCAAGAACTCTCAGATCTTTTCTACAGCTTCTGA
TAATCAACCAACTGTTACAATCAAGGTCTATGAAGGTAATTACCTTAAGTTTGGTTAATAT
CATGGCTTTTTTTTTGAGATGAAGTCTTGCTCTGTTGCCCAGGCTGGACTGCAGTGGCACG
ATCTCGGCTCACTGCAAATTCTGTCTCCCGGGTTCAAGTGATTCTCCTGCCTCAGCCTCCA
GAGTAGCTGGATTACAGCCTGACCACCACACCTGGCTAATTTCTGTATTTTTAGTAGAGGA
TGGGCTTTCACCATGTTTCCCAGGCTGGTCTCCAACTCCTGACCTCAGGTCATCTGCCTGC
CTCCACCGTCCCGAAAGTACTGGGATTATAGCGTGAGCCACCACGCCAGATCTATCTATCA
TGGCATATTTTAAAAGAACATGACTTAATATGTCCTATTGAAATGGCTAGGGAACTAAGTA
ACTGCTGTTTTCAGATGGAGGTCTTAATTTGAATAATGTTGATATTAGATATTTAGCATTC
TTTTTTTTTTTTTTTTAATGGAGTCTTGCTCTGTCGCCTAGGCTGGGGTGCAGTGGCATGA
CTTGCAACCTCTGCCTCCCGAATAGCTGGATTACAGGTGCCCACCATCACGCCCGGCTAA
GTTTTGTATTTTTAGTAGAGGCGAGTTTCGCCATGTTGGCCAGGCTGGTCTTGAACCCCTA
ACCTCAGTGATCCCACGGTCACCGACCTGGCCTCCCAAAAGTACTGTACCCAGCCAATGAT
TAGCATTCTCACTAATAATAGCATCTGAGCTGGCTCCTAGAGTACAAGAAAAAGGAGTTCA
CAGTACTTTAAAATAGATAAAATTCAGTTGAGTTAGTAACCTAACTCATTGTTAGTACTAG
TTGCTGCTCCTTGTAGACCAATATGAAATTACTTTTAGCTCGATAAAACCAAAAGTGTCAC

TTTATGCTTCAGACTGAAATGCGGGGATCTAGATGTGCTAATGCTTGTCAGTAACAACTAA

CAAGTTTTTCTGTATGTAACTTCTAGGTGAAAGACCCCTGACAAAGACAATCATCTTCTG

GGTACATTTGATCTGACTGGAATTCCTCCTGCTCCTCGTGGGGTCCCACAGATTGAAGTCA

CCTTTGAGATAGATGTGAATGGTATTCTTCGAGTGACAGCTGAAGACAAGGGTACAGGAA

CAAAAATAAGATCACAATCACCAATGACCAGAATCGCCTGACACCTGAAGAAATCGAAAGG

ATGGTTAATGATGCTGAGAAGTTTGCTGAGGAAGACAAAAAGCTGAAGGAGCGCATTGATA

CTAGAAATGAGTTGGAAAGCTATGCCTATTCTCTAAAGAATCAGATTGGAGATAAAGAAAA

GCTGGGAGGTAAACTTTCCTCTGAAGATAAGGAGACCATGGAAAAAGCTGTAGAAGAAAAG

ATTGAATGGCTGGAAAGCCACCAAGATGCTGACATTGAAGACTTCAAAGCTAAGAAGAAGG

AACTGGAAGAAATTGTTCAACCAATTATCAGCAAACTCTATGGAAGTGCAGGCCCTCCCCC

AACTGGTGAAGAGGATACAGCAGAAAAAGATGAGTTGTAGACACTGATCTGCTAGTGCTGT

AATATTGTAAATACTGGACTCAGGAACTTTTGTTAGGAAAAAATTGAAAGAACTTAAGTCT

CGAATGTAATTGGAATCTTCACCTCAGAGTGGAGTTGAAACTGCTATAGCCTAAGCGGCTG

TTTACTGCTTTTCATTAGCAGTTGCTCACATGTCTTTGGGTGGGGGGGAGAAGAAGAATTG

GCCATCTTAAAAAGCGGGTAAAAAACCTGGGTTAGGGTGTGTGTTCACCTTCAAAATGTTC

TATTTAACAACTGGGTCATGTGCATCTGGTGTAGGAGGTTTTTTCTACCATAAGTGACACC

AATAAATGTTTGTTATTTACACTGGTCTAATGTTTGTGAGAAGCTT

### GRP78 protein amino acid sequence (SEQ ID NO: 21):

MKLSLVAAMLLLLSAARAEEEDKKEDVGTVVGIDLGTTYSCVGVFKNGRVEIIAND
QGNRITPSYVAFTPEGERLIGDAAKNQLTSNPENTVFDAKRLIGRTWNDPSVQQDIKFLPF
KVVEKKTKPYIQVDIGGGQTKTFAPEEISAMVLTKMKETAEAYLGKKVTHAVVTVPAYFND
AQRQATKDAGTIAGLNVMRIINEPTAAAIAYGLDKREGEKNILVFDLGGGTFDVSLLTIDN
GVFEVVATNGDTHLGGEDFDQRVMEHFIKLYKKKTGKDVRKDNRAVQKLRREVEKAKRALS
SQHQARIEIESFYEGEDFSETLTRAKFEELNMDLFRSTMKPVQKVLEDSDLKKSDIDEIVL
VGGSTRIPKIQQLVKEFFNGKEPSRGINPDEAVAYGAAVQAGVLSGDQDTGDLVLLDVCPL
TLGIETVGGVMTKLIPRNTVVPTKKSQIFSTASDNQPTVTIKVYEGERPLTKDNHLLGTFD

LTGIPPAPRGVPQIEVTFEIDVNGILRVTAEDKGTGNKNKITITNDQNRLTPEEIERMVND

AEKFAEEDKKLKERIDTRNELESYAYSLKNQIGDKEKLGGKLSSEDKETMEKAVEEKIEWL

ESHQDADIEDFKAKKKELEEIVQPIISKLYGSAGPPPTGEEDTAEKDEL

The GRP78-like peptide according to the present invention may comprise a part of the GRP78 amino acid sequence represented by SEQ ID NO: 21 and may be a peptide comprising amino acid residues at which GRP78 phosphorylation or oxidation reactions may occur. The amino acid residues may preferably be selected from the group consisting of serine (S), threonine (T), tyrosine (Y), cysteine (C), methionine (M), tryptophan (W), glutamic acid (D), and proline (P). The GRP78-like peptide according to the present invention may comprise one or more, two or more, or preferably three or more of the amino acids described above. The peptide preferably has a length of 40-mer or less, 35-mer or less, 30-mer or less, or 25-mer or less.

The GRP78-like peptide according to the present invention may be a peptide comprising an amino acid sequence identical or similar to the NBD represented by SEQ ID NO: 22 or a part thereof. The part of the NBD may be, for example, the IA, IB, IIA, or IIB subdomain or a part thereof, but is not necessarily limited thereto. The similar amino acid sequence refers to an amino acid sequence having a sequence homology of at least 70%, at least 80%, at least 90%, or at least 95% with a reference amino acid sequence.

### 125-280 amino acid sequence of GRP78 (SEQ ID NO: 22):

KPYIQVDIGGGQTKTFAPEEISAMVLTKMKETAEAYLGKKVTHAVVTVPAYFNDAQ

RQATKDAGTIAGLNVMRIINEPTAAAIAYGLDKREGEKNILVFDLGGGTFDVSLLTIDNGV

FEVVATNGDTHLGGEDFDQRVMEHFIKLYKKKTGKDVRK

The GRP78-like peptide according to the present invention may be a peptide comprising an amino acid sequence identical or similar to the SBD represented by SEQ ID NO: 23 or a part thereof. The part of the SBD may be, for example, an alpha or beta subdomain or a part thereof, but is not necessarily limited thereto. The amino acid sequence similar to the SBD refers to an amino acid sequence having a sequence homology of at least 70%, at least 80%, at least 90%, or at least 95% with a reference amino acid sequence.

### 420-500 amino acid sequence of GRP78 (SEQ ID NO: 23):

CPLTLGIETVGGVMTKLIPRNTVVPTKKSQIFSTASDNQPTVTIKVYEGERPLTKD

NHLLGTFDLTGIPPAPRGVPQIEVT

The GRP78-like peptide according to the present invention may comprise an amino acid sequence having a sequence homology of at least 70%, at least 80%, at least 90%, at least 95%, or at least 100% with an amino acid sequence selected from the group consisting of SEQ ID NOs: 1 to 18 or a part thereof, but is not limited thereto.

**[Table 1]**

| Amino acid sequence of GRP78-like peptide | |
|---|---|
| **SEQ ID NO.** | **Amino acid sequence** |
| 1 | TFAPEEISAMVLTK |
| 2 | KPYIQVDIGGGQTKTFAPEEISA |

| | |
|---|---|
| 3 | MVLTKMKETAEAYLGKKVTH |
| 4 | MEHFIKLYKKKTGKDVRK |
| 5 | MEHRTRSWALGLSILGFFALLFSAGFVQQAHA |
| 6 | MEHRNRSWALGLSILGFFALLFSAGFVQQAHA |
| 7 | MDHRNRSWALGLSILGFFALLFSAGFVQQAHA |
| 8 | DAGTIAGLNVMR |
| 9 | AKFEELNMDLFR |
| 10 | AVVTVPAYFNDAQRQAT |
| 11 | KDAGTIAGLNVMRIINEPTAAA |
| 12 | IAYGLDKREGEKNILVFDLGGG |
| 13 | TFDVSLLTIDNGVFEV |
| 14 | VATNGDTHLGGEDFDQRV |
| 15 | CPLTLGIETVGGVMTKLIPR |
| 16 | NTVVPTKKSQIFSTASDNQP |
| 17 | TVTIKVYEGERPLTKDNHLL |
| 18 | GTFDLTGIPPAPRGVPQIEVT |

The peptides of SEQ ID NOs: 1, 8, and 9 are peptides having amino acid sequences predicted to be phosphorylated or oxidized within the entire GRP78 protein sequence based on protein structure and sequence analysis.

The peptides of SEQ ID NOs: 2 to 4 and 10 to 14 are peptides having amino acid sequences corresponding to parts of the NBD of GRP78. The NBD is divided into sequences IA, IB, IIA, and IIB. The peptides of SEQ ID NOs: 2 and 3 comprise amino acid sequences of IA, the peptides of SEQ ID NOs: 10 and 11 comprise amino acid sequences of IB, the peptides of SEQ ID NOs: 12 and 13 comprise amino acid sequences of IIA, and the peptides of SEQ ID NOs: 4 and 14 comprise amino acid sequences of IIB. These peptides recognize and bind to ATP, which is a substrate of GRP78.

The peptides of SEQ ID NOs: 15 to 18 are peptides having amino acid sequences corresponding to parts of the SBD of GRP78. The SBD is divided into the SBD alpha domain and the SBD beta domain. The peptides of SEQ ID NOs: 15 and 16 comprise the amino acid sequence of the SBD alpha domain, and the peptides of SEQ ID NOs: 17 and 18 comprise the amino acid sequence of the SBD beta domain. These peptides bind to peptides that bind to other substrates, which are breakdown products of ATP, which is regulated by GRP78.

Preferably, the GRP78-like peptide according to the present invention may comprise an amino acid sequence having a sequence homology of at least 70%, at least 80%, at least 90%, at least 95%, or at least 100% with an amino acid sequence selected from the group consisting of SEQ ID NOs: 1 to 18. More preferably, the GRP78-like peptide according to the present invention may comprise any one amino acid sequence selected from the group consisting of SEQ ID NOs: 1 to 18.

Preferably, the GRP78-like peptide according to the present invention may comprise an amino acid sequence having a sequence homology of at least 70%, at least 80%, at least 90%, at least 95%, or at least 100% with an amino acid sequence selected from the group consisting of SEQ ID NOs: 1 to 4. More preferably, the GRP78-like peptide according to the present invention may comprise any one amino acid sequence selected from the group consisting of SEQ ID NOs: 1 to 4.

The GRP78-like peptide according to the present invention is interpreted as comprising variants or fragments thereof in which an amino acid residue at a specific amino acid residue position is conservatively substituted, while having an amino acid sequence selected from the group consisting of SEQ ID NO: 1 to SEQ ID NO: 18. As used herein, the term "conservative substitution" refers to a modification of a peptide that involves the substitution of one or more amino acids with other amino acids having similar biochemical properties that do not result in loss of the biological or biochemical function of the corresponding peptide.

The term "conservative amino acid substitution" refers to substitution of the amino acid residue with an amino acid residue having a similar side chain. Families of amino acid residues having similar side chains have been defined and are well known in the art to which the present invention pertains. These families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine).

The GRP78 or GRP78-like peptide of the present invention has been found to retain the activity thereof in spite of conservative amino acid substitutions.

Without being bound by theory, the GRP78-like peptide of the present invention may comprise amino acid residues likely to undergo phosphorylation or oxidation of GRP78, comprise amino acid residues present within the NBD of GRP78, or comprise amino acid residues present within the SBD of GRP78, and thus modulate the inherent biochemical interactions of the GRP78 protein (e.g., interactions for regulating ATP and calcium homeostasis, binding to substrate proteins for protein unfolding regulation, etc.), thereby acting as a substance that mimics the effects of the GRP78 protein.

The active ingredient according to the present invention preferably increases GRP78 expression when delivered to cells. Here, increasing GRP78 expression includes directly or indirectly increasing GRP78 expression. Such a substance may be a peptide comprising an NBD represented by SEQ ID NO: 22 or a part thereof, an SBD represented by SEQ ID NO: 23 or a part thereof, or a part of the GRP78 amino acid sequence represented by SEQ ID NO: 21, as described above, and may be a peptide comprising three or more amino acids selected from the group consisting of serine (S), threonine (T), tyrosine (Y), cysteine (C), methionine (M), tryptophan (W), glutamic acid (D), and proline (P).

The peptide preferably has a length of 40-mer or less, 35-mer or less, 30-mer or less, or 25-mer or less.

When the active ingredient according to the present invention is a nucleic acid encoding GRP78, the nucleic acid may be DNA, mRNA, plasmid DNA, or the like, and may be delivered into cells using a cell-penetrating functional nanocarrier. The nucleic acid may be used in combination with various delivery vehicles, such as lipid nanoparticles (LNPs) and liposomes, which are known to effectively deliver oligonucleotides into cells, but are not limited thereto.

GRP78, a GRP78-like peptide, or a nucleic acid encoding the same according to the present invention may be useful for inhibiting or reversing aging of cells, tissues, or organs, or for restoring or regenerating the function of aged or damaged cells, tissues, or organs.

In the present invention, the cell, tissue, or organ may be a cell, tissue, or organ derived from a patient suffering from a disease or condition selected from the group consisting of muscle disease, bone disease, joint disease, skin disease, metabolic disease, urinary system disease, neurological disorder, cardiovascular disease, pulmonary dysfunction, cancer, immune disease, dental disease, periodontal disease, and oral tissue dysfunction. The patient may be an adult patient and may be, for example, 20 years or older, 30 years or older, 40 years or older, 50 years or older, 60 years or older, or 70 years or older.

Muscle mass declines after the age of 30 and is affected by decreased levels of growth hormone or androgen. Muscle diseases for which the GRP78 or GRP78-like peptide according to the present invention may be useful include, but are not limited to, sarcopenia, muscular atrophy, myasthenia, muscular dystrophy, myotonia, hypotonia, muscular weakness, muscular dystrophy, atony, amyotrophic lateral sclerosis, cachexia, masticatory muscle disorder, temporomandibular disorder, or inflammatory myopathy.

Bone loss (osteoporosis) typically begins in men in their 50s and in women after menopause. The elasticity of the ligaments connecting joints decreases and arthritis develops due to long-term cartilage wear and inflammation, also a common joint disease associated with aging. Bone or joint diseases for which the GRP78 or GRP78-like peptide according to the present invention may be useful include, but are not limited to, osteoporosis, Paget's disease, rickets, osteomalacia, osteolysis, renal osteodystrophy in patients with renal failure, fractures, degenerative bone diseases, osteogenesis imperfecta, osteopenia, bone defects and hip dysplasia, osteoarthritis, degenerative arthritis, osteochondritis dissecans, postmeniscal injury arthrosis or arthritis, joint malalignment, avascular necrosis, arthroses, isolated chondral defects, chondromalacia patellae, synovitis, bursitis, traumatic effusion, ligamentous deficiency arthroses, osteochondritis dissecans (OCD), patellar instability, rheumatoid arthritis, juvenile idiopathic arthritis, juvenile arthritis, post-traumatic arthritis, inflammatory arthritis, septic arthritis, lupus, scleroderma, tendonitis, fibrositis, fibromyositis, or polymyositis.

With aging, skin turnover increases and subcutaneous fat decreases. Vitamin D deficiency is common because the ability of the skin to synthesize vitamin D3 decreases. Skin becomes thinner, less elastic, drier, and more wrinkled. Other conditions associated with aging include cataracts, retinal degeneration, hearing loss, and decreased sensitivity of taste buds. Skin diseases for which the GRP78 or GRP78-like peptide according to the present invention may be useful include, but are not limited to, pruritus, seborrheic dermatitis, contact dermatitis, xeroderma, urticaria, acne, psoriasis, eczema, exfoliative dermatitis, hidradenitis, atopic dermatitis, drug allergy, diabetic dermatitis, bacterial dermatitis, fungal dermatitis, hidradenitis, allergic dermatitis, or neurodermatitis.

Metabolic diseases for which the GRP78 or GRP78-like peptide according to the present invention may be useful may include, but are not limited thereto, obesity, diabetes, hyperlipidemia, hypertriglyceridemia, liver disease, arteriosclerosis, stroke, myocardial infarction, cardiovascular disease, hyperglycemia, insulin resistance, or hyperinsulinemia.

As aging progresses, kidney weight and the number of glomeruli decrease, and glomerulosclerosis occurs. Furthermore, renal vascular resistance increases, leading to decreased blood flow to the kidney and a decreased glomerular filtration rate, which cause renal failure. The incidence of electrolyte abnormalities, glomerulonephritis, and chronic renal failure also increases. Urinary incontinence may occur due to decreased bladder storage capacity or weakened bladder muscles, and in men, an enlarged prostate is often present. Urinary system diseases for which the GRP78 or GRP78-like peptide according to the present invention may be useful may include, but are not limited to, urinary incontinence, cystitis, nephritis, benign prostatic hyperplasia, prostatitis, overactive cystitis, or urinary stones.

With increasing age, various neurological and brain diseases, such as depression, delirium, emotional and behavioral disorders, dementia, cerebrovascular disease, and Parkinson's disease are observed. With aging, the number and weight of brain neurons decrease by approximately 10%, and the area of the ventricles increases. Fatty brown pigment accumulation in neurons, amyloid deposition in cerebral blood vessels, senile plaques, and neurofibrillary tangles increase, and reactive oxygen species production increases, all of which are associated with nervous system damage. Furthermore, there is a decrease in enzymes and receptors involved in neurotransmitter production, as well as a decrease in cerebral blood flow and glucose metabolism. Neurological disorders for which the GRP78 or GRP78-like peptide according to the present invention may be useful may include, but are not limited thereto, spinal muscular atrophy, Friedreich's ataxia, CLN3 Batten, CLN6 Batten, CLN7 Batten, epileptic encephalopathy, Leigh syndrome, charcot-marie-tooth disease, giant axonal neuropathy, Lafora disease, SLC13A5 epileptic encephalopathy, congenital glycosylation disorder, Type IQ, Carriage syndrome, Angelman syndrome, Rett syndrome, spastic paraplegia, childhood alternating hemiplegia, or Zellweger spectrum disorder.

With aging, the prevalence of cardiovascular diseases such as hypertension, aortic disease, heart failure, ischemic heart disease, arrhythmias, peripheral vascular disease, and valvular disease increases. The heart exhibits left ventricular hypertrophy and left atrial dilatation with increasing age. This may be attributed to factors such as myocardial cell necrosis and enlargement, decreased growth hormone, and localized collagen deposition. Furthermore, vascular elasticity decreases, which is attributed to decreased endothelial cell production and a decrease in the vasodilator nitric oxide (NO) during the aging process. Cardiovascular diseases for which the GRP78 or GRP78-like peptide according to the present invention may be useful may include, but are not limited thereto, myocardial infarction, angina, hypertension, heart failure, cardiomyopathy, primary cardiac arrest, congestive heart disease, ischemic heart failure, arteriosclerosis, arrhythmia, coronary artery disease, stroke, or peripheral vascular disease.

Age-related lung disease is associated with decreased lung function, including weakened respiratory muscles and diaphragm, decreased elastic recoil of the lungs, and decreased pulmonary diffusing capacity (the rate of gas exchange between the lungs and blood). The increased risk of airway obstruction in the elderly increases mortality from chronic obstructive pulmonary disease, cardiovascular disease, and the incidence of lung cancer. Lung dysfunction diseases for which the GRP78 or GRP78-like peptide according to the present invention may be useful may include, but are not limited to, pulmonary hypertension, chronic obstructive pulmonary disease, pulmonary fibrosis, acute respiratory distress syndrome, bronchial asthma, inflammatory lung disease, pneumonia, or emphysema.

Cancer is sometimes referred to as a disease of aging. For example, the risk of developing cancer is known to increase more than tenfold after the age of 65. Aging may lead to a decrease in apoptosis, which may increase the risk of cancer. Cancers or immune-related diseases for which the GRP78 or GRP78-like peptide according to the present invention may be useful may include, but are not limited to, cancer, rheumatoid arthritis, insulin-dependent diabetes, juvenile diabetes, systemic lupus erythematosus, atopic dermatitis, Crohn's disease, psoriasis, multiple sclerosis, hyperthyroidism, anemia, autoimmune diseases including Behçet's disease and autoimmune encephalomyelitis, and transplant rejection diseases including graft-versus-host disease.

Aging occurs throughout the body, but in particular, aging of oral tissues, which play a crucial role in maintaining independent living, causes functional decline, tooth extraction, recession, and degenerative loss of function due to physiological decline in teeth, surrounding tissues, and secretory glands (such as salivary glands) and thus threatens the quality of life and lives of the elderly. Periodontal tissue damage due to diseases such as periodontitis, loss of oral muscles (masticatory muscles), and loss of teeth, gum bone, and masticatory function are known to be common diseases associated with old people. Furthermore, it is known that overall skeletal muscle loss with aging leads to various complications, including a decline in oral muscles (masticatory muscles), falls, fractures, and metabolic syndrome. Aging of oral tissues not only causes direct changes such as tooth loss, gingival tissue damage and inflammatory destruction, and degenerative changes in secretory tissues such as salivary glands, but also has direct and indirect effects on metabolic diseases such as heart disease, obesity, and diabetes, and neurological aging pathologies such as Alzheimer's and Parkinson's disease. Dental or periodontal diseases for which the GRP78 or a GRP78-like peptide according to the present invention may be useful may include, but are not limited to, gingivitis, periodontitis, gingival pain, gingival bleeding, severe bad breath, gingival discoloration, tooth loosening, tooth movement, gingival recession, gingival edema, periodontal pocket formation, pericoronitis, or periodontal abscess.

Dental or periodontal diseases for which the GRP78 or GRP78-like peptide according to the present invention may be useful may have, but are not limited to, impaired oral tissue function, which may include at least one selected from the group consisting of tooth wear, tooth loss, gingival recession, loss of periodontal ligament attachment, alveolar bone loss, and decreased saliva secretion, but are not necessarily limited thereto. Such a dental or periodontal disease may be selected from the group consisting of periodontitis, gingivitis, pulpitis, mucositis, exfoliative oral disorder, oral lichen planus, pemphigus vulgaris, stomatitis, root caries, alveolar bone defect, inflammatory fibrous hyperplasia, temporomandibular joint disorder, peri-implantitis, xerostomia, or oral cancer, but is not limited thereto.

Diseases or conditions for which the GRP78 or GRP78-like peptide according to the present invention may be useful may have aged muscle cells or muscle tissue, and may, for example, be accompanied by one or more characteristics selected from the group consisting of, but not limited to, muscle loss, muscle atrophy, muscle wasting, and muscle degeneration. Such a disease or condition may be selected from the group consisting of atony, muscular atrophy, muscular dystrophy, muscle degeneration, myotonia, amyotrophic lateral sclerosis (ALS), myasthenia gravis, cachexia, masticatory muscle disorder, temporomandibular disorder, and sarcopenia, but is not limited thereto.

The GRP78-like peptide according to the present invention, particularly a peptide comprising an NBD represented by SEQ ID NO: 22 or a part of an amino acid thereof, or a peptide comprising an SBD represented by SEQ ID NO: 23 or a part of an amino acid thereof, or a peptide comprising a part of a GRP78 amino acid sequence represented by SEQ ID NO: 21, wherein the peptide comprises three or more amino acids selected from the group consisting of serine (S), threonine (T), tyrosine (Y), cysteine (C), methionine (M), tryptophan (W), glutamic acid (D) and proline (P), or the peptide is capable of increasing the expression of GRP78, is considered to potentially reduce endoplasmic reticulum stress in a manner similar to the GRP78 protein that regulates endoplasmic reticulum stress, and thereby exhibits utility in inhibiting or reversing aging of cells, tissues or organs, or in restoring or regenerating the function of aging or damaged cells, tissues or organs. Therefore, the GRP78-like peptide according to the present invention, particularly a peptide comprising an NBD represented by SEQ ID NO: 22 or a part of an amino acid thereof, or a peptide comprising an SBD represented by SEQ ID NO: 23 or a part of an amino acid thereof, or a peptide comprising a part of the GRP78 amino acid sequence represented by SEQ ID NO: 21, wherein the peptide comprises three or more amino acids selected from the group consisting of serine (S), threonine (T), tyrosine (Y), cysteine (C), methionine (M), tryptophan (W), glutamic acid (D), and proline (P), or the peptide is capable of increasing the expression of GRP78, may be useful for the prevention or treatment of various diseases associated with endoplasmic reticulum stress through such a mechanism.

As used herein, the term "endoplasmic reticulum" refers to a crucial cellular organelle where protein folding occurs during protein biosynthesis, and plays a crucial role in maintaining cellular homeostasis. The endoplasmic reticulum stress response, similar to autophagy, is closely associated with cell survival or death.

The endoplasmic reticulum is a membrane structure in which branches extend from the nuclear envelope, and is classified into two types: rough endoplasmic reticulum having attached ribosomes and smooth endoplasmic reticulum having no ribosomes. Approximately one-third of intracellular proteins undergo post-translational modifications in the ER, including folding, assembly, glycosylation, and disulfide bond formation, to become active protein structures, after translation from mRNA to proteins. The smooth ER is also a site for the synthesis of lipids and steroid hormones and acts as a calcium storage unit, playing a crucial role in regulating intracellular calcium concentration.

As used herein, the term "endoplasmic reticulum stress (ER stress)" refers to a disruption in the ER function caused by the influx of immature proteins into the cell body beyond the capacity of the ER to process, due to physiological or pathological conditions, or by depletion of calcium within the ER. In other words, under ER stress, unfolded and abnormal proteins accumulate within the ER, potentially leading to various pathological conditions. Causes of ER stress include environmental changes in the cells due to diseases or the addition of chemical reagents, decreased glucose concentration, the addition of inhibitors of glycosylation enzymes, and the hyperphosphorylation of viral proteins due to viral infection. When ER stress occurs, cells develop a defense mechanism for survival thereof, which is referred to as "ER stress response".

The ER stress response is triggered by signals transmitted through ER transmembrane receptors located on the ER membrane: pancreatic ER kinase (PKR)-like ER kinase (PERK), activating transcription factor 6, and inositol-requiring enzyme 1 (IRE1). In normal cells, these three ER stress receptors are inactivated when binding to the ER chaperone GRP78/BiP, but, when unfolded proteins accumulate in the ER, GRP78/BiP is released from the receptors, activating the receptors and triggering the ER stress response. This ER stress response restores ER functions and reduces the accumulation of unfolded proteins. However, when the accumulation of unfolded proteins persists and ER stress is not alleviated, the apoptotic pathway is activated, ultimately leading to cell death.

The ER stress response is linked to mitochondrial function. For example, increased calcium release from the ER due to ER stress leads to mitochondrial dysfunction, including increased mitochondrial reactive oxygen species (ROS) production. Accordingly, the degree of ER stress may be determined by measuring changes in mitochondrial copy number and mitochondrial synthesis.

As used herein, the term "ER stress-related disease" refers to a disease caused by the accumulation of unfolded proteins within the ER due to the ER stress, including neurodegenerative diseases, chronic metabolic diseases, cancer, and viral infections.

Specifically, neurodegenerative diseases are caused by protein tangles that generally occur with the progression of aging, and endoplasmic reticulum stress is known to be frequently found in these diseases. Examples of the neurodegenerative diseases include Alzheimer's disease, Parkinson's disease, Huntington's disease, hypoxia of the brain and heart, cardiac hypertrophy, and arteriosclerosis. The relationship between the diseases described above and endoplasmic reticulum stress corresponds to the mechanism in Alzheimer's disease, wherein the PERK (PKR-like ER kinase) pathway, which plays an important role in the expression and regulation of the GRP78 protein, and the downstream substrate thereof, eIF2a (eukaryotic translation initiation factor 2 alpha) pathway are activated in the tissue samples of the patient, and endoplasmic reticulum stress is reduced by blocking protein synthesis within the cell as a whole by inactivating eIF2a. In Parkinson's disease, Parkin, an E3 ligase protein, is known to suppress cell death caused by endoplasmic reticulum stress, and endoplasmic reticulum stress itself is known to increase the expression of Parkin. In Huntington's disease, glutamine polymers formed when a part of a gene is over-amplified due to a mutation form protein tangles that are difficult for the cells to decompose. When this situation persists for a long time, endoplasmic reticulum stress occurs and nerve cell death is induced. Cerebral and cardiac hypoxia: hypoxia (ischemia) in the brain and heart disrupts cellular redox regulation, increases unfolded proteins, and induces endoplasmic reticulum (ER) stress. Furthermore, hypoxia in the brain and heart disrupts cellular redox regulation, increases unfolded proteins, and induces ER stress. Furthermore, reperfusion after hypoxia increases nitric oxide levels, which disrupts the balance of reactive oxygen species and s-nitrosylation within the cells, further increasing cellular oxidative stress.

In diabetes, which is a representative chronic metabolic disease, both type 1 and type 2 diabetes are known to be related to endoplasmic reticulum stress. In particular, in type 2 diabetes, stress enzymes (stress kinases) activated by endoplasmic reticulum stress are known to disrupt insulin-initiated cell signaling, rendering cells insensitive to insulin and preventing normal glucose uptake.

In addition to the diseases described above, endoplasmic reticulum (ER) stress is known to be involved in the development of various diseases, including immunoglobulin light chain amyloidosis, amyotrophic lateral sclerosis (ALS), hemodialysis-related amyloidosis, reactive amyloidosis, cystic fibrosis, sickle cell anemia, Creutzfeldt-Jakob disease, familial hypercholesterolaemia, alpha1 antitrypsin deficiency, cirrhosis, systemic emphysema, cerebral hereditary amyloidosis, Wolcott-Rallison syndrome, Wolfram syndrome, inflammatory bowel disease, Coronary artery disease, ulcerative colitis, breast cancer, and prostate cancer.

Therefore, it is suggested that modulating ER stress could prevent and treat the aforementioned ER stress-related diseases.

The GRP78 or GRP78-like peptide or nucleic acid encoding the same according to the present invention may be utilized in medical or cosmetic applications. In this regard, the term "composition" herein may refer to a pharmaceutical composition or a cosmetic composition.

In another aspect, the present invention is directed to a pharmaceutical composition for preventing or treating muscle diseases, comprising GRP78 or a GRP78-like peptide, or a nucleic acid encoding the same.

In another aspect, the present invention is directed to a method of preventing or treating muscle diseases comprising administering the peptide or nucleic acid.

In another aspect, the present invention is directed to the use of the peptide or nucleic acid for prevention or treatment of muscle diseases.

In another aspect, the present invention is directed to the use of the peptide or nucleic acid for the manufacture of a medicament for preventing or treating muscle diseases.

In the present invention, the muscle disease may be selected from the group consisting of sarcopenia, muscular atrophy, myasthenia, muscular dystrophy, myotonia, hypotonia, muscular weakness, muscular dystrophy, atony, amyotrophic lateral sclerosis, cachexia, masticatory muscle disorder, temporomandibular disorder, and inflammatory myopathy, but is not limited thereto.

The pharmaceutical composition of the present invention may comprise at least one pharmaceutically acceptable additive. The additive includes, but is not limited to, stabilizers, surfactants, lubricants, solubilizers, buffers, sweeteners, bases, adsorbents, glidants, binders, suspending agents, hardeners, antioxidants, polishing agents, flavoring agents, flavor enhancers, pigments, coating agents, wetting agents, wetting modifiers, fillers, defoamers, cooling agents, chewing agents, antistatic agents, colorants, sugar-coating agents, isomerization agents, softening agents, emulsifiers, tackifiers, thickeners, foaming agents, pH regulators, excipients, dispersants, disintegrants, waterproofing agents, preservatives, storage stabilizers, dissolution aids, solvents, and fluidizing agents

The pharmaceutical composition according to the present invention may further comprise a pharmaceutically acceptable carrier, excipient, and diluent, and examples of the pharmaceutically acceptable carrier, excipient, and diluent include, but are not limited to, those commonly used for preparation of drugs, including lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, syrup, methyl hydroxybenzoate, propyl hydroxybenzoate, talc, magnesium stearate, mineral oil and the like, but are not limited thereto.

The composition of the present invention may be prepared into an injectable formulation such as an aqueous solution, suspension, emulsion, or the like, a pill, a capsule, a granule, or a tablet. Therefore, the composition of the present invention may be an injection, a patch, a liquid, a capsule, a granule, a tablet, a powder, a spray, an ointment, a gel, a mucosal administration formulation, a suppository, or the like. These formulations may be prepared by a conventional method used for preparation in the art or by a method disclosed in Remington's Pharmaceutical Science (recent edition), Mack Publishing Company, Easton PA, and may be prepared into various formulations depending on each disease or ingredient.

The composition of the present invention may be administered orally or parenterally (e.g., intravenously, subcutaneously, intraperitoneally, or topically) depending on the intended method and the dosage range varies depending on the patient's weight, age, gender, health condition, diet, administration time, administration method, excretion rate, and severity of the disease.

In another aspect, the present invention is directed to a cosmetic composition for preventing or reversing aging, comprising GRP78, a GRP78-like peptide, or a nucleic acid encoding the same.

In the present invention, the composition may have at least one function selected from the group consisting of ameliorating skin wrinkles, changing skin or hair color, and alleviating or preventing hair loss and reversing such changes, but is not limited thereto.

The cosmetic composition of the present invention may be any cosmetic composition commonly used in the art and is preferably provided for use as a topical skin ointment, skin care cosmetic, makeup cosmetic, body cosmetic, or shaving cosmetic.

Furthermore, the cosmetic composition according to the present invention may be a cosmetic composition mixed with another cosmetic composition, specifically, skin lotion, skin softener, skin toner, astringent, lotion, milk lotion, moisturizing lotion, nourishing lotion, massage cream, nourishing cream, moisturizing cream, hand cream, essence, nourishing essence, pack, soap, shampoo, cleansing foam, cleansing lotion, cleansing cream, body lotion, body cleanser, emulsion, pressed powder, loose powder, eye shadow, or the like. The composition may be diluted as needed.

Furthermore, the cosmetic composition of the present invention may further comprise a functional substance having wrinkle-ameliorating or whitening effects, or, in one embodiment, a functional substance having UV-protecting or moisturizing effects, or excipients or diluents for improving physical properties, in addition to the peptide according to the present invention as an active ingredient. In one embodiment, the cosmetic composition may further comprise fragrances, pigments, bactericides, antioxidants, preservatives, moisturizers, thickeners, inorganic salts, or synthetic polymers to improve physical properties. Other compounding ingredients that may be added include oils, fats, emollients, surfactants, organic pigments, inorganic pigments, organic powders, ultraviolet absorbers, pH adjusters, alcohols, blood circulation promoters, cooling agents, antiperspirants, purified water or the like.

Hereinafter, the present invention will be described in more detail with reference to examples. However, it will be obvious to those skilled in the art that these examples are provided only for illustration of the present invention and should not be construed as limiting the scope of the present invention.

### Example 1: Evaluation of reversion potential of aged cells to younger cells by inducing increased GRP78 expression in aged cells

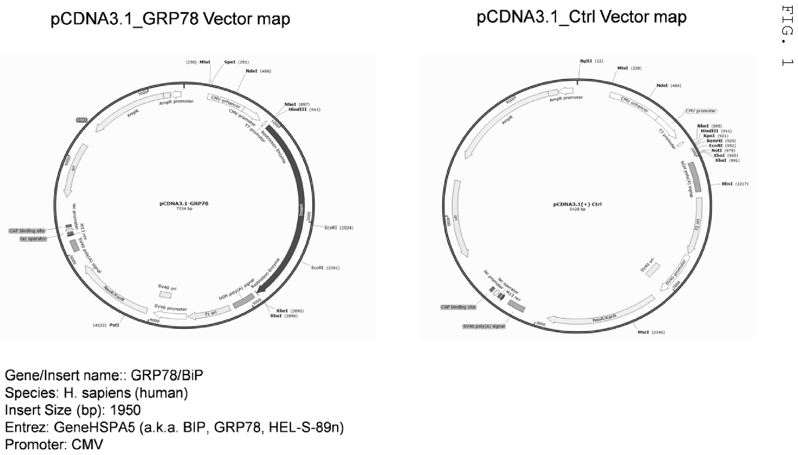

Experiments were conducted using a GRP78 overexpression vector including the nucleic acid sequence of SEQ ID NO: 20. The GRP78 gene was overexpressed in aged model stem cells. 72 hours after transfection, GRP78 overexpression in cells and changes in aging-related and stem cell-potentiation-related gene expression depending on GRP78 overexpression were determined using qRT-PCR. The constructed vector is shown in FIG. 1.

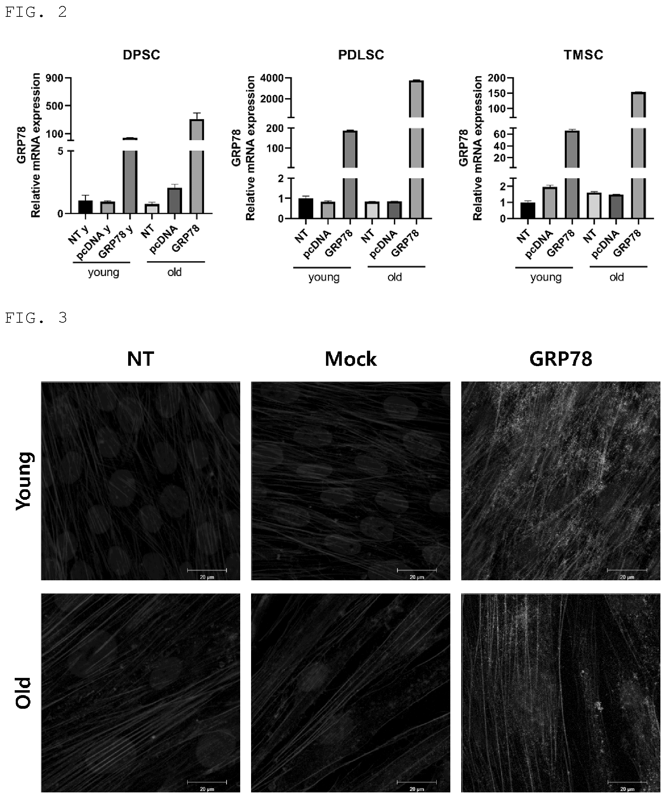

The experimental results showed that, when the GRP78 expression vector was introduced into both young cells and aged cells, GRP78 mRNA expression levels significantly increased. Specifically, compared to the control group in which the GRP78 vector was not introduced, the expression level of mRNA in the experimental group in which the GRP78 vector was introduced increased by a minimum of 60-fold and a maximum of 4,000-fold (FIG. 2). These results are consistent with the results observed by FACS in Example 2 below, which showed that more vectors were introduced into aged cells when comparing the gene introduction efficiency between young and aged cells, which indicates that the GRP78 overexpression vector was effectively introduced into aged cells.

### Example 2: Evaluation of molecular genetic changes through introduction of anti-aging factor into cells

To evaluate molecular genetic changes after introducing the pcDNA3.1 vector containing the GRP78 gene, which was found to function as an anti-aging factor in Example 1, into stem cells, experiments were performed using a fluorescently labeled GRP78-EGFP expression vector provided by Chungbuk National University. Young stem cells (10 passages or less) and aged stem cells (15 passages or more) were established through subculture of DPSCs. The vector was then introduced into each cell using a transfection reagent. 72 hours after transfection, the cells were fixed and fluorescently stained. Whether or not the introduced GRP78-EGFP was expressed was determined using confocal microscopy. The result of confocal microscopy showed that fluorescence was observed in cells transfected with only the GRP78-EGFP vector (FIG. 3), which indicates the normal introduction of the GRP78-EGFP vector into the cells. Furthermore, both young and aged cells were equally transfected with the genes.

Furthermore, to determine the efficiency of gene expression vector transfection in young and aged cells, additional analysis was performed using FACS. The GRP78-EGFP expression vector was transfected into young or aged DPSCs. 72 hours after transfection, the cells were completely detached from the cell culture plate with trypsin-EDTA and clumped cells were filtered using a FASC strainer. For FACS analysis, non-treated cells were gated and analyzed using a 488 nm fluorescence wavelength.

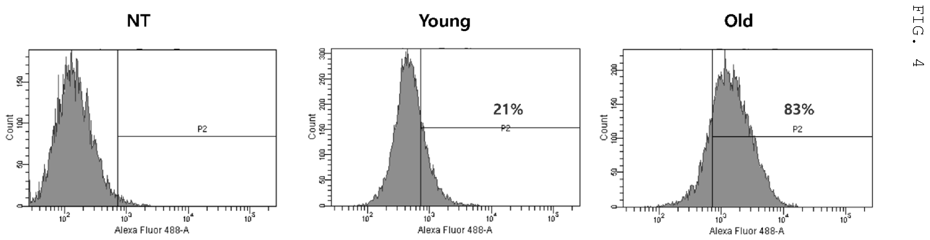

As a result, approximately 21% of cells expressed GRP78-EGFP when introduced into young cells. This indicates that the vector transfection efficiency in young stem cells was approximately 21%. On the other hand, the result of analysis of aged stem cells showed that approximately 83% of the cell population exhibited fluorescence. The GRP78 expression vector had higher transfection efficiency in aged stem cells than in young stem cells, and normal protein expression was also observed in aged DPSCs (FIG. 4).

In addition, to determine GRP78 mRNA expression induced by the intracellularly transfected GRP78 vector, the GRP78 expression vector was transfected into dental stem cells (DPSCs and PDLSCs). 72 hours later, qPCR was performed using Sybr green to determine the amount of TERT mRNA, a factor involved in the length of telomere expressed.

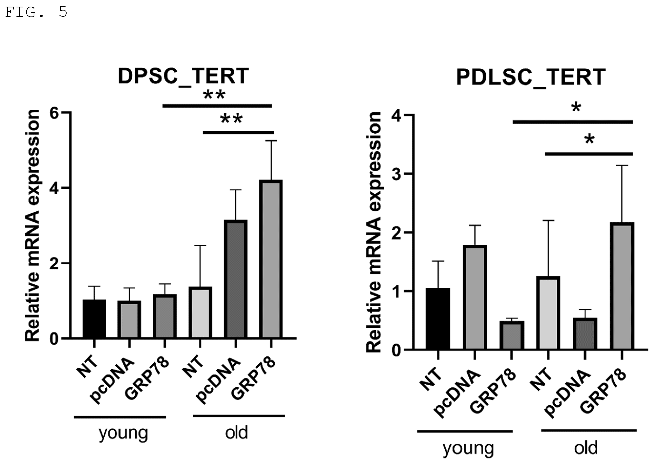

As a result, the introduced GRP78 vector significantly increased TERT expression in aged DPSCs and PDLSCs compared to young DPSCs and PDLSCs (FIG. 5, **p<0.01 compared to young GRP78 and old NT in DPSCs, respectively; *p<0.05 compared to young GRP78 and old NT in PDLSCs, respectively). This suggests that the expression of GRP78 protein introduced into aged cells significantly increased the expression of TERT as an aging marker and that GRP78 also has an aging-reversing effect (FIG. 5).

### Example 3: Evaluation of tissue changes by introduction of anti-aging factors into aging animal model

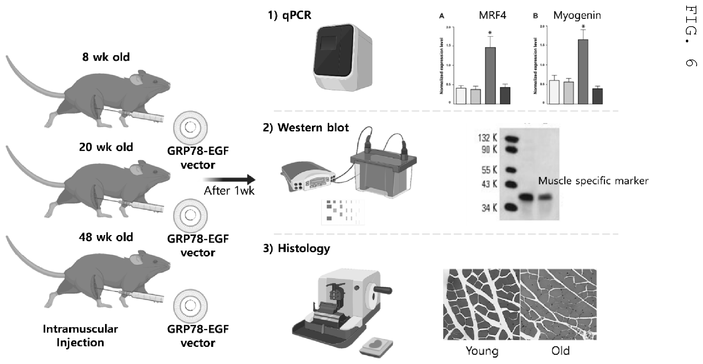

To evaluate tissue changes by the introduction of anti-aging factors in an aging animal model, experiments were performed to determine the increased mRNA and protein levels of GRP78 after the introduction of a GRP78 expression vector at the cellular level. To determine whether or not tissue delivery of the GRP78 expression vector also increases GRP78 mRNA and protein expression in tissues, the GRP78 expression vector was transfected into the gastrocnemius muscle of mice using an *in vivo* transfection reagent. The experimental groups were divided into three groups: young (8-week-old) mice, middle-aged (20-week-old) mice, and naturally aged (48-week-old) mice (FIG. 6). 50 µl of a mixture of the transfection reagent and the vector was injected into each gastrocnemius muscle.

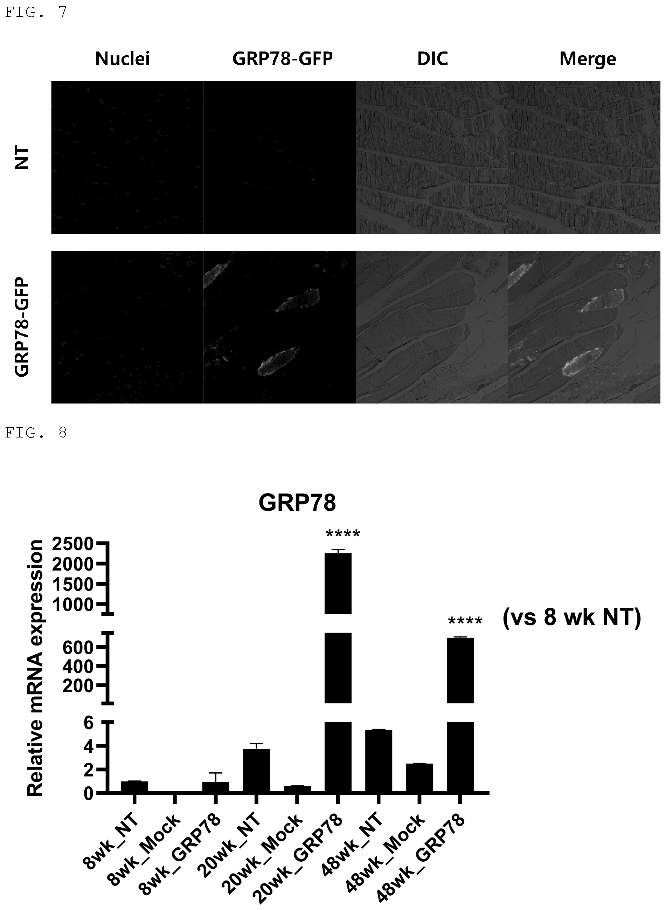

First, to determine tissue transfection with the vector, transfection reagent-vector was injected into the gastrocnemius muscle using the GRP78-EGFP expression vector. 72 hours after injection, mice were sacrificed to obtain gastrocnemius muscles. Cryosectioning was performed using an OCT compound. Nuclear staining of the tissue was performed with DAPI and was observed by confocal microscopy. As a result, fluorescence was observed in the gastrocnemius muscles of animals injected with the GRP78-EGFP vector, which indicates the successful injection of the GRP78 expression vector using the transfection reagent (FIG. 7).

7 days after injection of the GRP78 expression vector into the gastrocnemius muscle of mice using a transfection reagent, in order to measure the mRNA and protein expression levels of GRP78 in the tissue, for GRP78 mRNA, total RNA was obtained from the tissue using TRIzol. The mRNA was reverse-transcribed to synthesize cDNA. GRP78 mRNA expression was quantified using the synthesized cDNA. The result showed that GRP78 RNA expression significantly increased, compared to the non-treated experimental group (FIG. 8; ****p<0.0001, compared to 8-week-old NT mice).

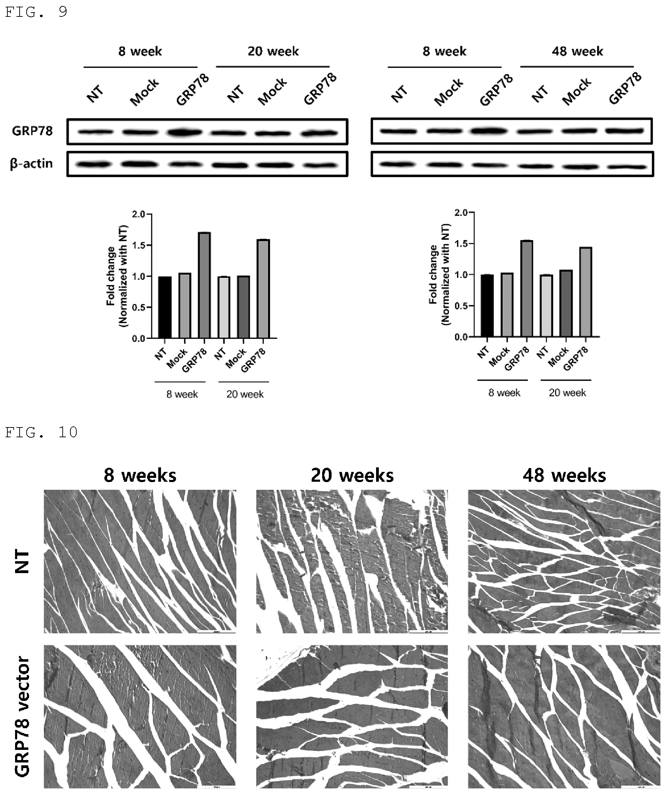

Furthermore, in order to determine the protein expression level of GRP78 in the tissue, gastrocnemius muscle lysates were obtained using RIPA buffer and Western blot analysis was performed. The result showed that protein expression increased by 50% or less in the GRP78 expression vector-injected group, compared to the other experimental groups (FIG. 9).

In addition, to histologically determine changes in muscle fascicle thickness, tissue sections were prepared using gastrocnemius muscle. To prepare tissue sections, the tissue was dehydrated and substituted with paraffin, and the tissue was embedded in paraffin. After embedding, 5 µm sections were produced and stained with hematoxylin and eosin (H&E).

The experimental results showed that the GRP78 expression vector-injected animals exhibited an increased muscle fascicle thickness, compared to the control group at each age, in particular, an increased muscle fascicle thickness even when compared to the 8-week-old control group (FIG. 10).

### Example 4: Synthesis of GRP78-like peptides

The peptides of SEQ ID NOs: 1 to 18 in Table 1 were synthesized sequentially from the N-terminus using F-moc solid phase peptide synthesis. The synthesized peptide sequences were cleaved from the resin, washed, lyophilized, and then isolated and purified by liquid chromatography. The molecular weight of the purified peptides was measured using MALDI-TOF analysis.

### Example 5: Comparison of expression of aging-related biomarkers

Rat periodontal ligament (rPDL) tissue cells were seeded in 60 mm culture dishes using MEM alpha modified 1X culture medium at a total of 4.5 × 10⁴ cells and then cultured. The required number of cells for the experiment was obtained, the culture medium was removed, the residue was washed twice with DPBS, and compared under a light microscope. SA-β gal staining was performed using a senescence β-galactosidase staining kit #9860 from cell signaling technology. Quantitative RT-PCR was performed using a magnetic induction cycler (Mic qPCR) from Bio Molecular Systems. Real-time cell proliferation curves were measured using the Incucyte^{®} S3 live-cell imaging and analysis instrument from Satorius Co. Ltd.

Changes in cell characteristics with aging in periodontal ligament tissue-derived cells from young rats (11 weeks old, control group) and aged rats (96 weeks old, aged group) were observed (FIG. 11). FIG. 11A shows the results of cell length and width measurements in the control and aged groups. Cell length significantly increased with aging (**p<0.01).

FIG. 11B shows the results of observation of the expression of beta-galactosidase, which is highly expressed in aged cells, using SA-β gal staining (increased blue staining). Beta-galactosidase expressed in the cells was stained using a senescence β-galactosidase staining kit, dissolved in 70% glycerol for 30 minutes, and measured for absorbance at 405 nm. As the number of aged cells increased, the degree of Beta-galactosidase staining increased, resulting in a significant increase in absorbance (***p<0.001).

FIG. 11C shows the results of RT-PCR of the gene expression levels of aging-related biomarkers: telomere length-related factor-1 (TRF-1), telomere length-related factor-2 (TRF-2), protection of telomeres 1 (POT1), and glucose-regulated protein 78 (GRP78). All four genes exhibited a significant decrease in expression in the aged group. The expression of TRF-1, TRF-2, and POT1 was significantly reduced in the aged group to 0.63 ± 0.18, 0.27 ± 0.03, and 0.31 ± 0.21, respectively, compared to the control group (***p<0.001). GRP78 expression also significantly decreased to 0.56 ± 0.38 in the aged group (**p<0.01).

FIG. 11D shows the results of cell proliferation measurements. The control group had 100% cell confluency on day 5 after seeding 5.3 × 10⁴ cells. On the other hand, the aged group had a proliferation rate of approximately 72% on day 5 due to aging. This indicates that the proliferation rate of periodontal ligament cells extracted from aged rats (old rPDL) is significantly lower than that of periodontal ligament cells extracted from young rats (young rPDL) (***p<0.001). Even at day 8, the end point of the experiment, the cell confluency of old rPDL was 82%.

These results show age-specific aging-related phenomena in rPDL cells, supporting the potential thereof as model cells for aging reversal.

### Example 6: Cell penetration of GRP78-like peptides

The peptides of SEQ ID NOs: 1 to 4 were labeled with Rhodamine B fluorescence. A total of 5.0 × 10⁴ cells were seeded, and the culture medium was removed and the cells were washed twice with DPBS. The prepared fluorescent peptides were mixed with serum-free culture medium at a concentration of 200 µg/mL and incubated for 1 hour. After 1 hour of treatment, the cells were washed five times with DPBS and mounted with a mounting solution containing DAPI. The intracellular penetration and distribution of the peptides were observed using an inverted confocal microscope.

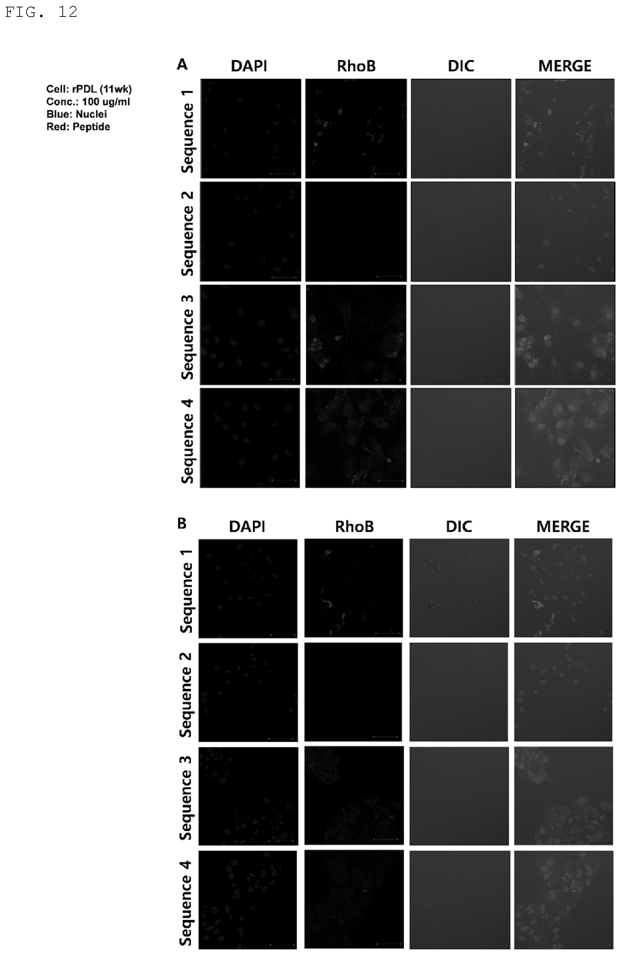

FIG. 12 shows the results of confocal microscopy of the cell penetration of the peptides of SEQ ID NOs: 1 to 4. FIG. 12A shows results of the cell permeability of periodontal ligament cells (young rPDL) extracted from young rats, and FIG. 12B shows results of the cell permeability of periodontal ligament cells (old rPDL) extracted from aged rats. The peptides having SEQ ID NOs: 1, 3, and 4 exhibited high cell permeability in both young and old rPDL, and the peptides exhibited lower permeability in old rPDL than in young rPDL. Therefore, it was found that aged cells exhibited reduced cell permeability compared to young cells.

### Example 7: Evaluation of aging reversal potential by GRP78-like peptides

To determine the aging reversal potential of GRP78-like peptides, the expression of aging factors by the peptides was compared. A total of 4.5 × 10⁴ tonsil-derived stem cells and rat periodontal ligament cells were seeded, the culture medium was removed and the cells were washed twice with DPBS. The four prepared peptide candidates were mixed at a concentration of 200 µg/ml in serum-free culture medium and incubated for 1 hour. After treatment for 1 hour, the cells were washed twice with DPBS, RNA was obtained and the expression of each gene was evaluated and compared using qRT-PCR.

FIG. 13 shows the results of measurement of changes in the expression of aging-associated biomarkers induced by peptides of SEQ ID NOs: 1 to 4.

FIG. 13A shows the results of RT-PCR to determine changes in the expression of aging-associated biomarkers TRF-1, TRF-2, and hTERT (human telomerase reverse transcriptase) induced by peptides of SEQ ID NOs: 1 to 4 in 20 passages of serially cultured aged human tonsil-derived mesenchymal stem cells (TMSCs). All aging-associated biomarker genes exhibited a significant decrease in expression in aging cells. GRP78 gene expression was also significantly reduced in aging cells.

TRF-1 expression significantly increased in aged cells treated with the peptides of SEQ ID NO: 1 and SEQ ID NO: 2, with a fold increase of 1.66±0.30 and 4.32±0.51, respectively, compared to non-treated cells (***p<0.001).

TRF-2 expression increased significantly in aged cells treated with the peptide SEQ ID NO: 1, with a fold increase of 2.86±0.10, compared to non-treated aged cells (***p<0.001). Treatment with the peptide SEQ ID NO: 3 significantly increased the expression by a fold increase of 1.23±0.04, compared to non-treated aged cells (*p<0.05).

For hTERT expression, treatment with the peptide of SEQ ID NO: 2 significantly increased hTERT expression by a fold increase of 2.53±0.48, compared to non-treated aged cells (***p<0.001). Treatment with the peptide of SEQ ID NO: 3 significantly increased hTERT expression by a fold increase of 2.21±0.16, compared to non-treated aged cells (*p<0.05).

FIG. 13B shows the results of RT-PCR to determine changes in the expression of aging-related biomarkers TRF-1, TRF-2, POT1, and GRP78 in periodontal ligament tissue-derived cells extracted from young (12-week-old) and aged (96-week-old) rats treated with the peptides of SEQ ID NOs: 1 to 4. Overall, the expression of all genes was significantly reduced in aged rat cells, compared to young rat cells ($p<0.001, comparison between Young rPDL_control and Old rPDL_control).

TRF-1 expression significantly increased in the young rPDL group by 1.34±0.12 and 1.21±0.11 folds, respectively, when treated with peptides SEQ ID NOs: 2 and 4 (***p<0.001). In the old rPDL group, treatment with peptides SEQ ID NOs: 2 and 4 also significantly increased the expression by 0.83±0.28 and 0.78±0.18 folds, respectively, compared to non-treated old rPDL (0.58±0.01) (***p<0.001).

TRF-2 expression significantly increased in the young rPDL group by 1.25±0.10 and 1.31±0.10 folds, respectively, when treated with peptides SEQ ID NOs: 2 and 4 (**p<0.01). In the old rPDL group, treatment with peptides SEQ ID NOs: 2, 3, and 4 also significantly increased TRF-2 expression by 0.51±0.21, 0.38±0.11, and 0.74±0.22 fold, respectively, compared to non-treated old rPDL (0.13±0.01) (***p<0.001).

POT1 expression significantly increased in the old rPDL group when treated with peptides of SEQ ID NOs. 2 and 4, with 0.34±0.20 (p<0.05) and 0.66±0.31 (**p<0.01) fold, respectively, compared to the non-treated old rPDL control group (0.25±0.01).

GRP78 expression significantly increased in the old rPDL group when treated with peptides of SEQ ID NOs: 2 and 4, with 0.52±0.18 (p<0.05), 0.65±0.04 (p<0.001), and 0.69±0.30 (p<0.01) fold, respectively, compared to the non-treated old rPDL group (0.56±0.01).

Overall, the peptides of SEQ ID Nos: 1 to 4 were demonstrated to be able to re-increase the expression of aging-related biomarker genes that have been reduced due to aging.

### Example 8: Evaluation of muscle tissue function recovery by peptides

To determine the ability of peptides to restore muscle function, muscle function was evaluated using a natural aging mouse model. The mice used herein were C57BL/6J male, and the ages thereof were 8 weeks (0.16 years, young), 48 weeks (1 year, middle-aged), and 72 weeks (1.5 years, old). Peptides of SEQ ID NOs: 3 and 4 were injected intramuscularly into the gastrocnemius muscle of the hindlimbs at a dose of 10 mg/kg, three times a week for four weeks. To evaluate muscle function recovery by peptides of SEQ ID NOs: 3 and 4, grip strength tests and rotarod tests were performed before drug injection and for eight weeks after drug injection. The grip strength test was performed to measure changes in the grip strength of the forelimbs of mice using a grip strength meter (JD-A-22, Jeung Do Bio and Plant Co., Ltd.). The rotarod test was performed to measure the time the mice could remain on a rotating rod without falling using a rotarod (JD-A-07TS, Jeung Do Bio and Plant Co., Ltd.) at a speed of 25 rpm. The time spent on the rod was measured for up to 8 weeks after administration.

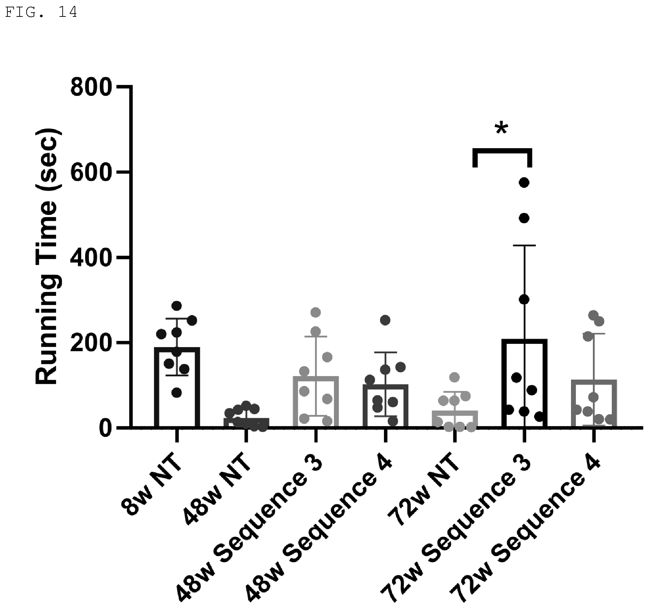

After 8 weeks, 48-week-old and 72-week-old mice administered the peptides of SEQ ID NOs: 3 and 4 exhibited no significant difference in rod retention time compared to 8-week-old (8 w NT) mice. Compared to 48-week-old mice not administered the peptides (48 w NT), 48-week-old mice administered the peptides of SEQ ID NOs: 3 and 4 exhibited an increase in rod retention time. Compared to 72-week-old mice not administered peptides (72 w NT), 72-week-old mice administered peptides of SEQ ID NO: 3 and SEQ ID NO: 4 exhibited an increased retention time on the rod, particularly 72-week-old mice administered with peptides of SEQ ID NO: 3 exhibited an increased retention time on the rod (*p<0.05, FIG. 14). Statistical significance between groups was analyzed using one-way ANOVA. These results indicate that peptides of SEQ ID NO: 3 and SEQ ID NO: 4 restored muscle function in aged mice.

### Example 9: Histological comparison of muscle fibers in naturally aged mice treated with peptides

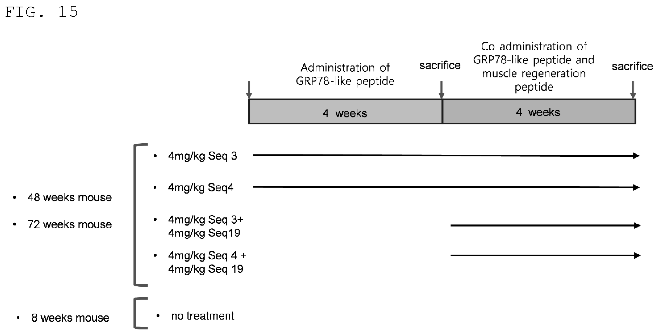

To determine the muscle tissue functional recovery ability of GRP78-like peptides, muscle function was evaluated using a natural aging mouse model. The mice used herein were C57BL/6J male mice and the ages thereof were 8 weeks (0.16 years, young), 48 weeks (1 year, middle-aged), and 72 weeks (1.5 years, old). The peptides of SEQ ID NO: 3 or SEQ ID NO: 4 were injected intramuscularly into the gastrocnemius muscle of the hind limbs of mice at a dose of 4 mg/kg, three times a week for 4 weeks. Subsequently, for an additional 4 weeks, 4 mg/kg of the peptides of SEQ ID NO: 3 or SEQ ID NO: 4, a combination of 4 mg/kg of the peptide of SEQ ID NO: 3 and 4 mg/kg of the muscle regeneration peptide (GKMSTRGRKMMRRKK: SEQ ID NO: 19), or , a combination of 4 mg/kg of the peptide of SEQ ID NO: 4 and 4 mg/kg of the muscle regeneration peptide (SEQ ID NO: 19) were injected intramuscularly into the gastrocnemius muscle of the hindlimbs of mice three times a week. Mice of each age group were sacrificed after 4 and 8 weeks, and tissue sections were prepared and stained with hematoxylin and eosin (H&E) to evaluate muscle tissue recovery. The type, dose, administration period, and sacrifice date of the peptides administered to each age group are shown in Table 2 and FIG. 15.

**[Table 2]**

| Peptide type, dose, administration period, and sacrifice date for mouse at each age | | | | |
|---|---|---|---|---|
| **Age** | **Treatment** | **Dose (mg/kg)** | **Treatment period** | **Sacrifice day** |
| 8 weeks | No treatment (NT) | 0 | 0 | 0 |
| 48 weeks | NT | 0 | 0 | 0 |
| | Seq 3 | 4 | 8 | 4 weeks, 8 weeks |
| | Seq 4 | 4 | 8 | 4 weeks, 8 weeks |
| | Seq 3+Seq 19 | 4+4 | 8 | 8 weeks |
| | Seq 4+Seq 19 | 4+4 | 8 | 8 weeks |
| 72 weeks | NT | 0 | 0 | 0 |
| | Seq 3 | 4 | 8 | 4 weeks, 8 weeks |
| | Seq 4 | 4 | 8 | 4 weeks, 8 weeks |
| | Seq 3+Seq 19 | 4+4 | 8 | 8 weeks |
| | Seq 4+Seq 19 | 4+4 | 8 | 8 weeks |

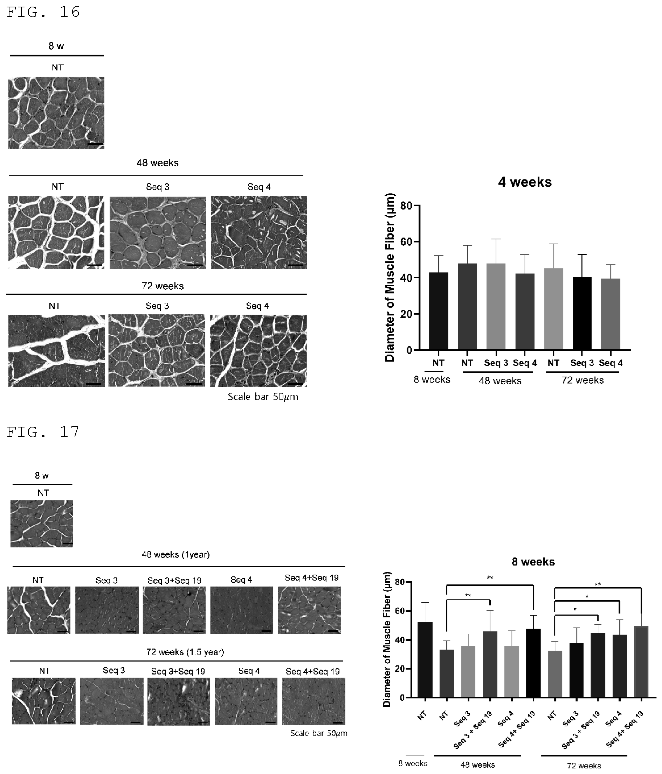

FIG. 16 shows the results of H&E staining and muscle fiber diameter measurements of muscle tissue from mice administered peptides of SEQ ID NO: 3 or SEQ ID NO: 4 for 4 weeks.

Compared to 8-week-old mice not administered peptides, the 48- and 72-week-old mice, which had naturally aged without peptide administration, exhibited an increase in the void between muscle fibers. In other words, the diameter of muscle fibers decreased with aging. On the other hand, 48- and 72-week-old mice administered peptides of SEQ ID NO: 3 or SEQ ID NO: 4 exhibited a decrease in the void between muscle fibers. Furthermore, the diameter of muscle fibers was similar to that of 8-week-old mice (FIG. 16). Therefore, it was found that the GRP78-like peptide of the present invention may reverse aging.

FIG. 17 shows the results of H&E staining and muscle fiber diameter measurements of mouse muscle tissues injected with peptides of SEQ ID NO: 3 or SEQ ID NO: 4 for 4 weeks, followed by an additional 4 weeks of administration of the peptide of SEQ ID NO: 3 or SEQ ID NO: 4 alone, or a combination of the peptide of SEQ ID NO: 3 or SEQ ID NO: 4 and a muscle regeneration peptide of SEQ ID NO: 3 or SEQ ID NO: 4.

Compared to 8-week-old mice not administered peptides, the void between the muscle fibers of naturally aged 48-week-old and 72-week-old mice not administered peptides increased. When the muscle fiber diameters of each tissue were measured, they decreased. Similar to the results in FIG. 16, the muscle fiber diameters were found to decrease in older cells.

In 48-week-old mice, muscle fiber diameter increased when administered only the peptides of SEQ ID NO: 3 or SEQ ID NO: 4 compared to when not administered any peptides. Furthermore, co-administration of SEQ ID NO: 3 with the muscle regeneration peptide and SEQ ID NO: 4 with the muscle regeneration peptide resulted in a more significant increase (*p<0.05). Furthermore, in 72-week-old mice, muscle fiber diameter increased when administered only the peptides of SEQ ID NO: 3 or SEQ ID NO: 4 compared to no peptide administration. Furthermore, co-administration of SEQ ID NO: 3 with the muscle regeneration peptide and SEQ ID NO: 4 with the muscle regeneration peptide resulted in a more significant increase (*p<0.05) (FIG. 17).

When the peptide of SEQ ID NO: 3 or 4 was administered alone, muscle density and muscle fiber diameter in naturally aged mice increased. When the peptide of SEQ ID NO: 3 or 4 was administered in combination with a muscle regeneration peptide, the effect of increasing muscle density and muscle fiber diameter in naturally aged mice was even greater.

### Example 10: Comparison of muscle biomarker expression by peptides

The tissue sections prepared in Example 9 were subjected to immunofluorescence (IF) staining using MyoD1 antibody (Ab16148, Abcam) and myosin heavy chain (MYH) antibody (sc-376157, Santa Cruz Biotechnology). The tissue sections obtained after paraffin-embedding were deparaffinized in xylene, sequentially immersed in 100%, 95%, and 70% ethanol to hydrate the tissue, and then rinsed in distilled water. The slides were immersed in a citrate buffer-based antigen retrieval buffer (pH 6.0) and then heat-treated at 95°C for 15 minutes to perform antigen retrieval. After antigen retrieval, the slides were cooled to room temperature and washed three times for 5 minutes each with PBS. The slides were blocked in a PBS solution containing 5% bovine serum albumin (BSA) for 1 hour at room temperature. The slides were treated with MYH and MyoD1 primary antibodies, each diluted to 1:200, followed by incubation at 4°C for 16 hours. After washing three times with PBS for 5 minutes, the slides were treated with a secondary antibody containing a fluorescent label (A-31570, Thermo Fisher Scientific), diluted to 1:500, followed by incubation at room temperature in the absence of light for 1 hour. The slides were washed three times with PBS for 5 minutes, stained with DAPI (D1306, Thermo Fisher Scientific) for 5 minutes, and then washed again with PBS. The slides were then mounted using fluorescent mounting medium (F4680, Sigma-Aldrich) and mounted with a coverslip.

MyoD1 is a marker expressed early in muscle regeneration and MYH is a marker expressed late in muscle regeneration (Montano, M. "Translational models, methods, and concepts in studies of muscle tissue repair" Translational Biology in Medicine (2014): 103-128)

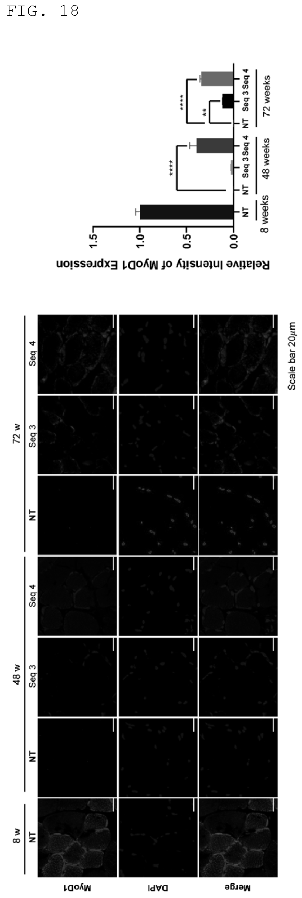

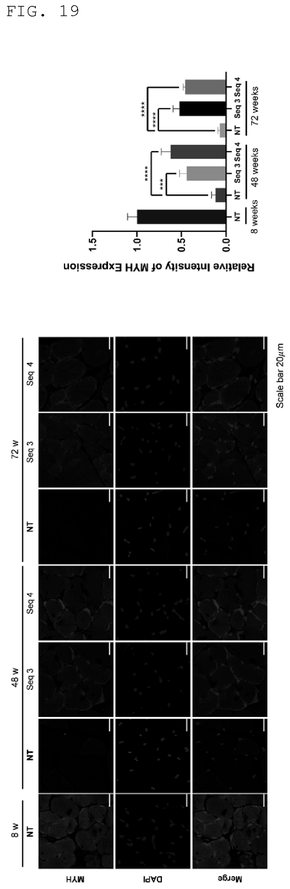

FIG. 18 shows the results of confocal microscopy to determine MyoD1 expression and fluorescence intensity measurements in muscle tissue from mice administered peptides of SEQ ID NO: 3 and SEQ ID NO: 4 for 4 weeks. FIG. 19 shows the results of confocal microscopy to determine MYH expression and fluorescence intensity measurements in muscle tissue from mice administered peptides of SEQ ID NO: 3 and SEQ ID NO: 4 for 4 weeks.

The 8-week-old mice not administered peptide exhibited the highest MyoD1 and MYH protein expression in muscle tissue. The 48-week-old mice administered SEQ ID NO: 3 or SEQ ID NO: 4 exhibited increased MyoD1 and MYH protein expression compared to 48-week-old mice not administered peptide. The 72-week-old mice administered SEQ ID NO: 3 or SEQ ID NO: 4 exhibited increased MyoD1 and MYH protein expression, compared to 72-week-old mice not administered the peptide (FIGS. 18 and 19).

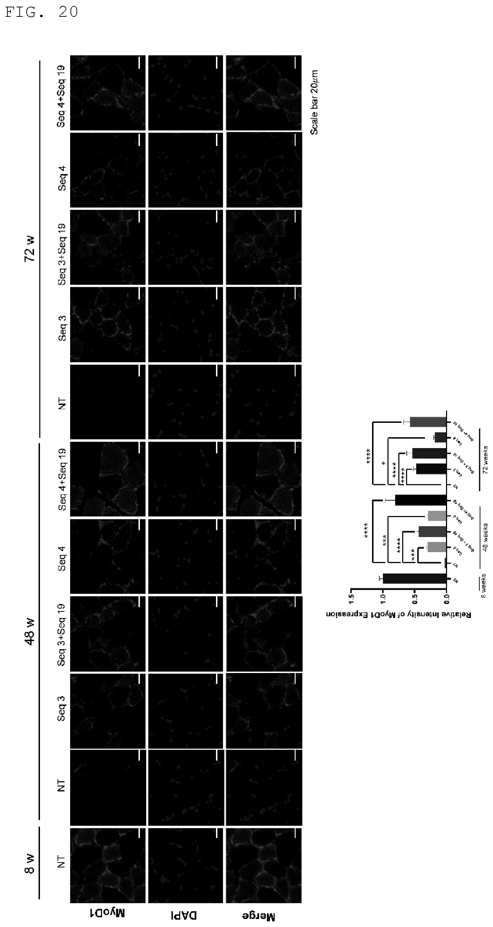

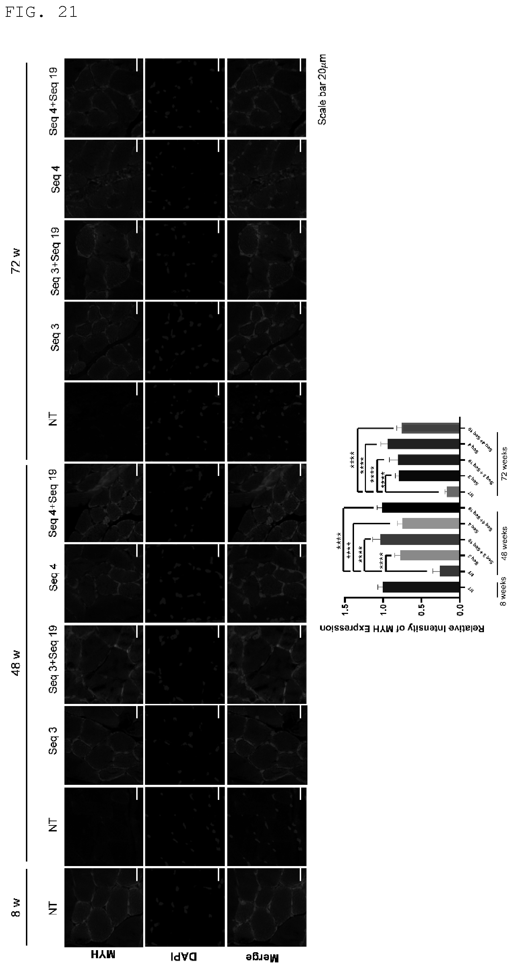

FIG. 20 shows the results of confocal microscopy to determine MyoD1 expression and fluorescence intensity measurements in the muscles of mice that administered the peptide of SEQ ID NO. 3 or SEQ ID NO. 4 alone for 4 weeks, and then further administered the peptide of SEQ ID NO. 3 or SEQ ID NO. 4 for 4 weeks, or administered a combination of the peptide of SEQ ID NO. 3 and a muscle regeneration peptide (SEQ ID NO: 19), or administered a combination of the peptide of or SEQ ID NO. 4 and a muscle regeneration peptide (SEQ ID NO: 19). FIG. 21 shows the results of confocal microscopy to determine the expression of MYH and fluorescence intensity measurement in the muscles of mice that administered the peptide of SEQ ID NO. 3 or SEQ ID NO. 4 alone for 4 weeks, and then further administered the peptide of SEQ ID NO. 3 or SEQ ID NO. 4 for 4 weeks, or administered a combination of the peptide of SEQ ID NO. 3 and a muscle regeneration peptide (SEQ ID NO: 19), or administered a combination of the peptide of or SEQ ID NO. 4 and a muscle regeneration peptide (SEQ ID NO: 19).

The highest muscle tissue expression levels of MyoD1 and MYH were observed in 8-week-old mice that had not been administered peptides. When 48-week-old mice administered peptides of SEQ ID NO: 3 or SEQ ID NO: 4, MyoD1 and MYH protein expression levels increased compared to 48-week-old mice that had not been administered peptides. Furthermore, administration of the combination of the peptide of SEQ ID NO: 3 and the muscle regeneration peptide, or administration of the combination of the peptide of SEQ ID NO: 4 and the muscle regeneration peptide significantly increased the expression of MyoD1 and MYH, compared to administration of the peptides alone (**p<0.01). In mice administered the combination of the peptide of SEQ ID NO: 3 and the muscle regeneration peptide, or the combination of the peptide of SEQ ID NO: 4 and the muscle regeneration peptide, MyoD1 and MYH expression was higher than in the youngest 8-week-old mice not administered the peptides (FIGS. 20 and 21).

In 72-week-old mice, MyoD1 and MYH protein expression increased when administered the peptide of SEQ ID NO: 3, the combination of the peptide of SEQ ID NO: 3 and the muscle regeneration peptide, or the combination of SEQ ID NO: 4 and the muscle regeneration peptide, compared to 72-week-old mice not administered the peptides. MyoD1 expression levels were similar when administered the peptide of SEQ ID NO: 3 alone or the combination of the peptide of SEQ ID NO: 3 and the muscle regeneration peptide. However, MYH expression levels increased more when administered the combination of the peptide of SEQ ID NO: 3 and the muscle regeneration peptide. MyoD1 expression levels increased more when administered the combination of the peptide of SEQ ID NO: 4 and the muscle regeneration peptide than when administered the peptide of SEQ ID NO: 4 alone. On the other hand, MYH expression levels remained similar.

Administration of the peptide of SEQ ID NO: 3 or SEQ ID NO: 4 alone increased the expression of muscle differentiation biomarkers in naturally aged mice. Co-administration of the peptide of SEQ ID NO: 3 or SEQ ID NO: 4 with the muscle regeneration peptide further enhanced the increase in muscle biomarker expression in naturally aged mice.

### Example 11: Evaluation of aging reversal effect through restoration of telomere length regulators

To evaluate the aging reversal effect of the GRP78-like peptide of the present invention, experiments were conducted on young TMSC (passage 5) and TMSC (passage 15) that had undergone replicative aging through subculture, the changes in various telomere-related regulatory factors closely related to aging, and amelioration, the aging reversal effect, and the restoration of telomere length regulators by GRP78-like peptide administration were also evaluated.

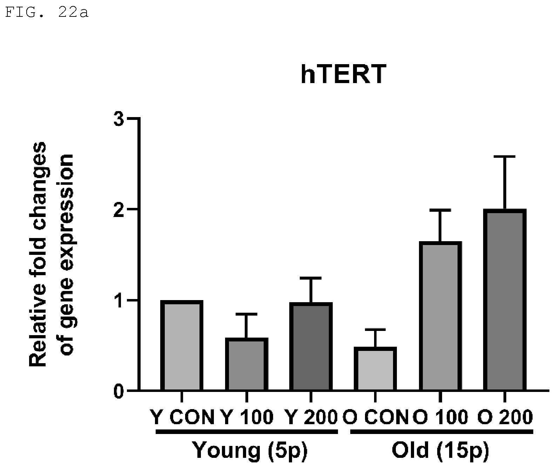

3.5 × 10⁵ TMSC were seeded in a 60 mm² dish, the culture medium was removed and the cells were washed twice with DPBS. The cells were then treated with the prepared GRP78 peptide (SEQ ID NO: 4) solution for 24 hours and samples were collected. Specific primers were synthesized using the nucleotide sequence of hTERT and qRT-PCR was performed using these primers. The results showed that hTERT expression decreased in the aged TMSC control group compared to young TMSC and administration of the peptide of SEQ ID NO. 4 restored hTERT expression (FIG. 22A).

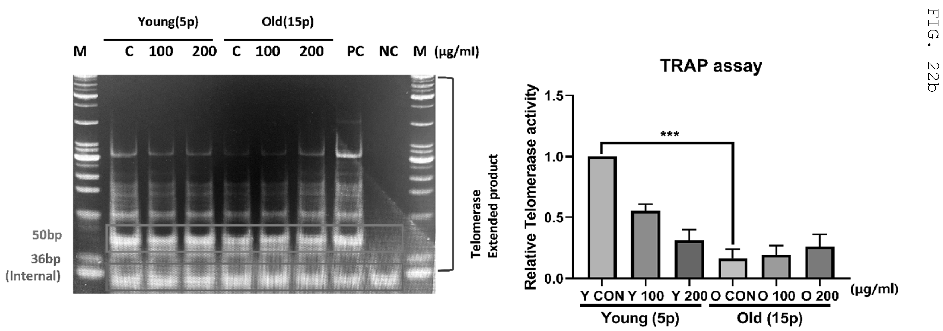

Furthermore, 3.5 × 10⁵ TMSCs were seeded on a 60 mm² dish, and cell culture and peptide treatment were performed in the same manner as in the experiment shown in FIG. 22A. The experiment was then performed using the TRAPeze^{™} RT telomerase detection kit in accordance with the instructions of Sigma-Aldrich. As a result, telomerase expression was significantly reduced in the aged TMSC control group compared to young TMSC (***p<0.001) and telomerase expression was increased when treated with the peptide of SEQ ID NO: 4 (FIG. 22B).

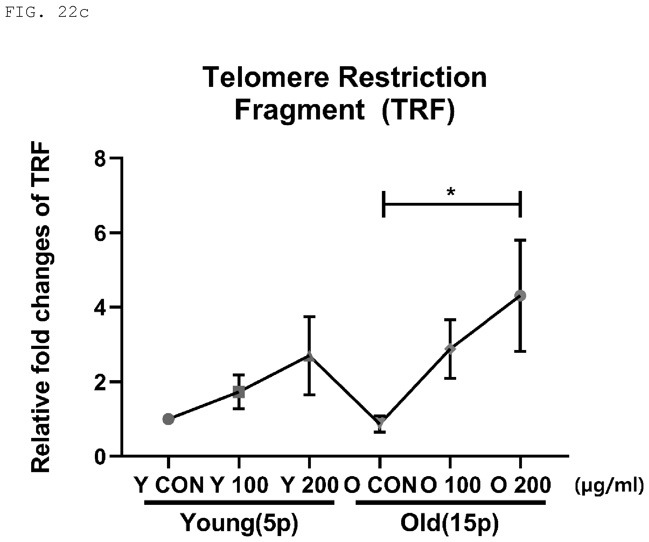

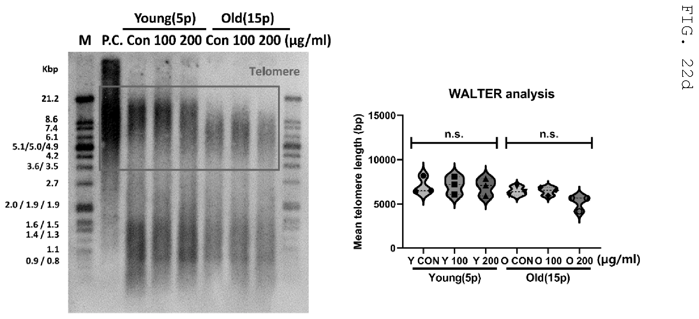

Next, 3.5 × 10⁵ TMSCs were seeded on a 60 mm² dish and cultured for 2 days until the cell confluency reached 70 to 80%. The TMSCs were treated with serum-free culture medium and GRP78 peptide (SEQ ID NO: 4) at various concentrations (0, 100, 200 µg/ml) for 24 hours. Then, the TeloTAGGG^{™} telomere length assay was performed in the same manner as the method suggested by Sigma-Aldrich. As a result, the telomere length in the control group of aged TMSCs was reduced by 0.87-fold compared to young TMSCs, and when aged TMSCs were treated with the peptide of SEQ ID NO: 4, the telomere length was significantly increased by 4.94-fold compared to the aged control group (*p<0.05) (FIG. 22C). Furthermore, visible telomere length was measured using southern blot analysis. The result showed that replicatively aged TMSCs had slightly shorter telomeres than young TMSCs (FIG. 22D).

Overall, the results show that the GRP78-like peptide of the present invention restores the expression of direct telomere length-related factors that were reduced due to replicative aging in TMSCs caused by passage culture, which indicates the ability of the GRP78-like peptide to improve or reverse aging.

### Example 12: Evaluation of effects of GRP78-like peptide on recovery of mitochondrial-related factors

The following experiments were conducted to evaluate the direct quantitative changes in mitochondria and related factors in TMSCs treated with the GRP78-like peptide of the present invention.

3.5 × 10⁵ TMSCs were seeded on a 60 mm² dish, the culture medium was removed, and the cells were washed twice with DPBS. The cells were treated with the prepared GRP78 peptide (SEQ ID NO: 4) solution and cultured for 24 hours. The medium was removed and the cells were fixed with 4% paraformaldehyde. After treatment with a mixture of phenol-free DMEM and 1 mM stock mitotracker for 30 minutes, the distribution of mitochondria in the TMSCs was observed using an inverted confocal microscope.

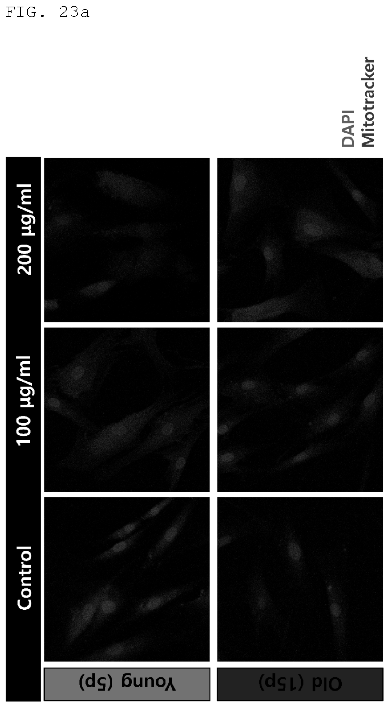

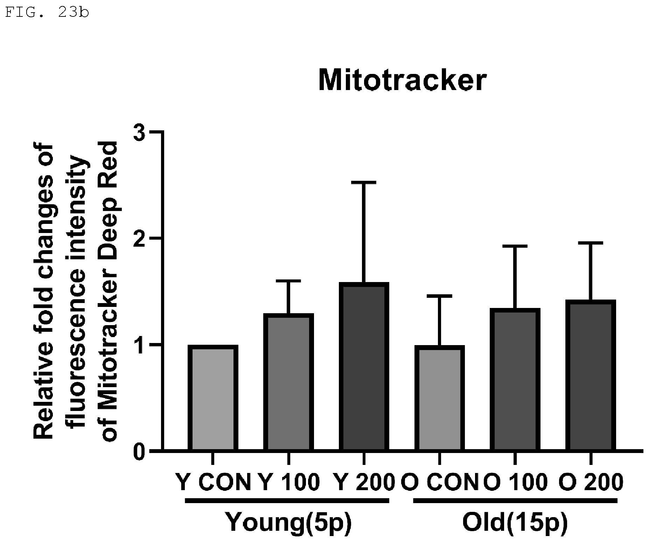

As a result, compared to young TMSCs, mitochondrial expression was observed to be relatively low in replicatively aged TMSCs, as determined using Mitotracker. Treatment with the peptide (SEQ ID NO: 4) exhibited a slight recovery in mitochondrial expression (FIGS. 23A and 23B).

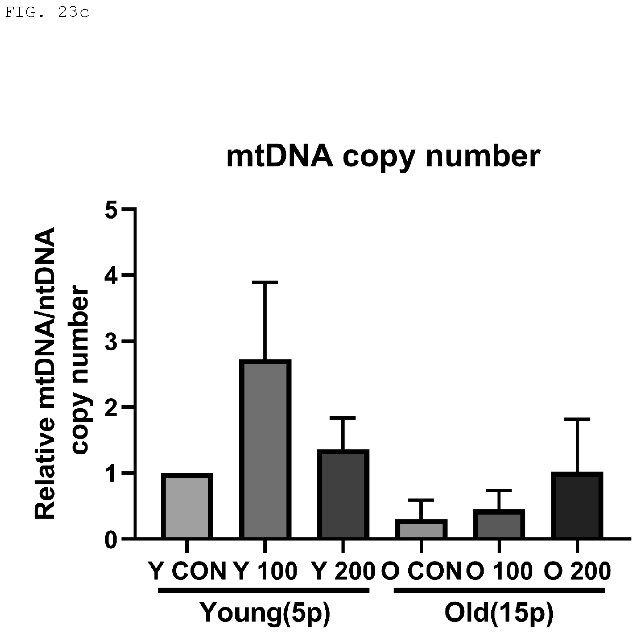

To determine quantitative changes in mitochondrial DNA (mtDNA), the change in mtDNA copy number was measured using qRT-PCR. Compared to young TMSCs, replicatively aged TMSCs had a relatively lower mtDNA copy number. Treatment with the peptide of SEQ ID NO: 4 significantly increased the copy number (FIG. 23C).

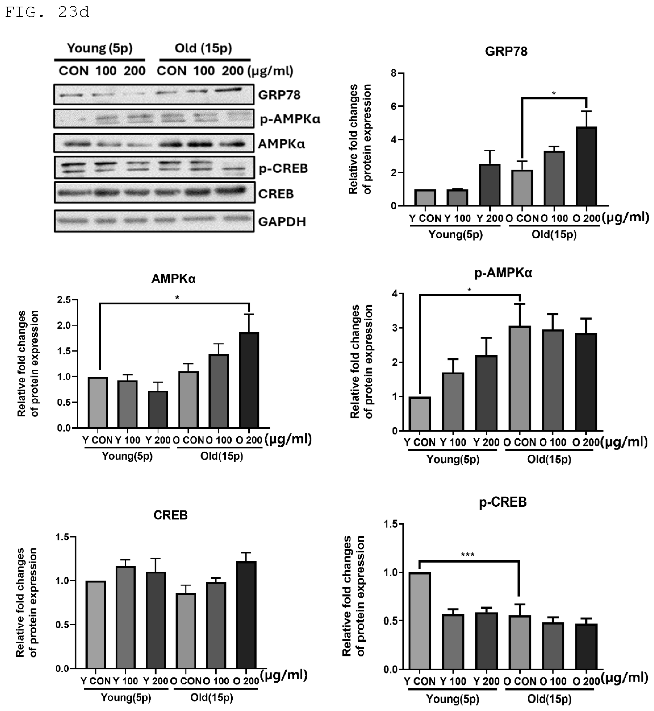

Furthermore, changes in protein expression of factors related to mitochondrial activity and biogenesis were determined. The result showed that treatment with the peptide of SEQ ID NO: 4 significantly increased direct GRP78 protein expression (*p<0.05). Treatment of replicatively aged TMSCs with the peptide also resulted in a relative increase in AMPKα and CREB expression compared to the replicatively aged control group (FIG. 23D).

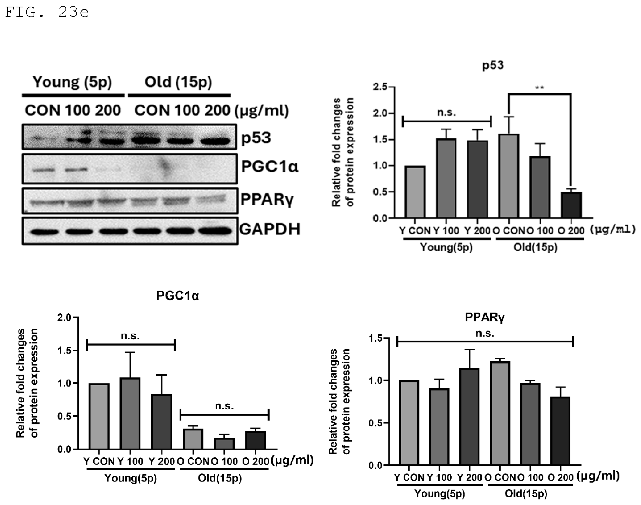

Furthermore, gene and protein expression changes induced by treatment of factors associated with mitochondria and telomeres such as PPARγ, PGC1α, and p53, with the peptide of SEQ ID NO: 4 were evaluated. The result showed that, compared to young TMSCs, p53 expression was higher in replicatively aged TMSCs (***p<0.001). Treatment with the peptide of SEQ ID NO: 4 at a concentration of 200 µg/ml significantly reduced p53 expression in replicativly aged TMSCs (**p<0.01) (FIG. 23E).

Based on the various experimental results related to mitochondria, treatment with the peptide of SEQ ID NO: 4 restored mitochondrial activity in replicatively aged TMSCs. Specifically, this suggests that the peptide may play a role in aging reversal and recovery by altering the expression of telomere-related factors. This indicates that the peptide of SEQ ID NO: 4 regulates ER stress response in a manner similar to the GRP78 protein, which potentially regulates ER stress, and has the function of restoring mitochondrial dysfunction and ultimately reversing and restoring aging.

### Industrial Applicability

The GRP78, GRP78-like peptide or nucleic acid encoding the same according to the present invention can increase the expression of a biomarker whose expression is reduced in aging cells, and thus is useful for restoring the function of muscle tissue or the like whose function is reduced due to aging.

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

### Sequence Listing Free Text

An electronic file is attached.

## Claims

1. A composition comprising GRP78, a GRP78-like peptide, or a nucleic acid encoding the same as an active ingredient, for inhibiting or reversing aging in cells, tissues, or organs, or restoring or regenerating the functions of aged or damaged cells, tissues, or organs.

2. The composition according to claim 1, wherein the active ingredient is a peptide comprising an amino acid sequence having a sequence homology of at least 80%, at least 90%, at least 95%, or at least 100% with a nucleotide binding domain represented by SEQ ID NO: 22 or a part thereof.

3. The composition according to claim 1, wherein the active ingredient is a peptide comprising an amino acid sequence having a sequence homology of at least 80%, at least 90%, at least 95%, or at least 100% with a substrate binding domain represented by SEQ ID NO: 23 or a part thereof.

4. The composition according to claim 1, wherein the active ingredient is a peptide comprising a part of a GRP78 amino acid sequence represented by SEQ ID NO: 21,
wherein the peptide has a length of 40-mer or less and comprises at least three amino acids selected from the group consisting of serine (S), threonine (T), tyrosine (Y), cysteine (C), methionine (M), tryptophan (W), glutamic acid (D), and proline (P).

5. The composition according to claim 1, wherein the active ingredient increases expression of GRP78.

6. The composition according to claim 1, wherein the active ingredient is a peptide comprising an amino acid sequence having a sequence homology of at least 70%, at least 80%, at least 90%, at least 95%, or at least 100% with an amino acid sequence selected from the group consisting of SEQ ID NOs: 1 to 18 or a part thereof.

7. The composition according to claim 1, wherein the active ingredient is a peptide comprising an amino acid sequence having a sequence homology of at least 70%, at least 80%, at least 90%, at least 95%, or at least 100% with an amino acid sequence selected from the group consisting of SEQ ID NOs: 1 to 4 or a part thereof.

8. The composition according to claim 1, wherein the active ingredient is a peptide comprising an amino acid sequence of SEQ ID NO: 1.

9. The composition according to claim 1, wherein the active ingredient is a peptide comprising an amino acid sequence of SEQ ID NO: 2.

10. The composition according to claim 1, wherein the active ingredient is a peptide comprising an amino acid sequence of SEQ ID NO: 3.

11. The composition according to claim 1, wherein the active ingredient is a peptide comprising an amino acid sequence of SEQ ID NO: 4.

12. The composition according to claim 1, wherein the cell, tissue, or organ is a cell, tissue, or organ derived from a patient suffering from a disease or condition selected from the group consisting of muscle disease, bone disease, joint disease, skin disease, metabolic disease, urinary system disease, neurological disorder, cardiovascular disease, pulmonary dysfunction, cancer, immune disease, dental disease, periodontal disease, and oral tissue dysfunction.

13. The composition according to claim 1, wherein the composition is used to restore or regenerate the function of aged muscle tissue.

14. The composition according to claim 13, wherein the aged muscle tissue is accompanied by decreased muscle function, muscle loss, muscle atrophy, muscle wasting or muscle degeneration.

15. The composition according to claim 13, wherein the composition is used to prevent or treat a muscle disease selected from the group consisting of sarcopenia, muscular atrophy, myasthenia, muscular dystrophy, myotonia, hypotonia, muscular weakness, muscular dystrophy, atony, amyotrophic lateral sclerosis, cachexia, masticatory muscle disorder, temporomandibular disorder, and inflammatory myopathy.

16. A cosmetic composition for preventing or reversing aging, comprising GRP78, a GRP78-like peptide, or a nucleic acid encoding the same.

17. The cosmetic composition according to claim 16, wherein the composition has at least one function selected from the group consisting of ameliorating skin wrinkles, changing skin or hair color, and alleviating or preventing hair loss.
